# EUROPÄISCHE PATENTSCHRIFT

(11) **EP 0 344 682 B1**
(45) Veröffentlichungstag und Bekanntmachung des Hinweises auf die Patenterteilung: **18.11.1993**
(21) Anmeldenummer: 89109647.1
(22) Anmeldetag: 29.05.1989
(51) Int. Cl.: C07K 5/00, A61K 37/64

(54) **Oligopeptide mit zyklischen Prolin-analogen Aminosäuren**
Oligopeptides with cyclic analogues of amino acids of proline
Oligopeptides contenant des analogues cycliques d'amino-acides de proline

(30) Priorität: 03.06.1988 DE 3818850
(43) Veröffentlichungstag der Anmeldung: 06.12.1989
(73) Patentinhaber: HOECHST AKTIENGESELLSCHAFT, 65926 Frankfurt am Main (DE)
(72) Erfinder: Henke, Stephan, Dr., D-6232 Bad Soden am Taunus (DE); Brocks, Dietrich Dr., D-6200 Wiesbaden (DE); Günzler-Pukall, Volkmar, Dr., D-3550 Marburg-Cappel (DE); Kivirikko, Kari Ilkka, SF-90230 Oulu (FI); Myllylä, Raili Mirja Helena, SF-90230 Oulu (FI)

(56) Entgegenhaltungen:
- EP-A- 0 190 852
- EP-A- 0 254 354
- EP-A- 0 271 865
- EP-A- 0 322 068
- WO-A-81/02893
- WO-A-86/02353
- JOURN. CHEM. SOC. PERKIN I, 1972, Seiten 2475-2481; R. FAIRWEATHER et al.: "Sequential polypeptides. Part VI. The synthesis of some sequential polypeptide collagen models containing proline analogues"
- THE JOURNAL OF BIOLOGICAL CHEMISTRY, Band 263, Nr. 36, 25. Dezember 1988, Seiten 19498-19504, The American Society for Biochemistry and Molecular Biology, Inc., Baltimore, MD, US; V. GÜNZLER et al.: "Syncatalytic inactivation of prolyl 4-hydroxylase by synthetic peptides containing the unphysiologic amino acid 5-oxaproline"

## Beschreibung

Die Erfindung betrifft Oligopeptide mit zyklischen, unnatürlichen Aminosäuren, die wirksame Inhibitoren der Prolylhydroxylase sind.

2,3-Disubstituierte Isoxazolidine und ihre Verwendung als Inhibitoren der Prolylhydroxylase sind in der deutschen Patentanmeldung Nr. 36 43 012.9 beschrieben.

Der Erfindung liegt die Aufgabe zugrunde, neue wirksame Inhibitoren der Prolylhydroxylase zu finden, deren Prolin-Rest durch zyklische, unnatürliche Aminosäuren ersetzt ist.

Diese Aufgabe wird gelöst durch die Verbindungen der Formel I 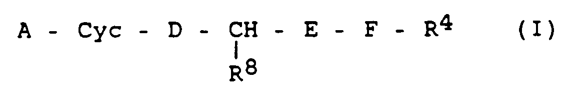
in welcher
A
   a₁)
      (C₁-C₈)-Alkyl,
      (C₁-C₈)-Alkanoyl,
      (C₁-C₈)-Alkoxycarbonyl oder
      (C₁-C₈)-Alkylsulfonyl bedeutet,
      in welchen jeweils 1, 2 oder 3 Wasserstoffatome gegebenenfalls durch 1, 2 oder 3 gleiche oder verschiedene Reste aus der Reihe
      Carboxy,
      Amino,
      (C₁-C₄)-Alkylamino,
      Hydroxy,
      (C₁-C₄)-Alkoxy,
      Halogen,
      Di-(C₁-C₄)-alkylamino,
      Carbamoyl,
      Sulfamoyl,
      (C₁-C₄)-Alkoxycarbonyl,
      (C₆-C₁₂)-Aryl und
      (C₆-C₁₂)-Aryl-(C₁-C₅)-alkyl ersetzt sind, oder in welchen jeweils 1 Wasserstoffatom gegebenenfalls durch einen Rest aus der Reihe
      (C₃-C₈)-Cycloalkyl,
      (C₁-C₄)-Alkylsulfonyl,
      (C₁-C₄)-Alkylsulfinyl,
      (C₆-C₁₂)-Aryl-(C₁-C₄)-alkylsulfonyl,
      (C₆-C₁₂)-Aryl-(C₁-C₄)-alkylsulfinyl,
      (C₆-C₁₂)-Aryloxy,
      (C₃-C₉)-Heteroaryl und
      (C₃-C₉)-Heteroaryloxy
      und
      1 oder 2 Wasserstoffatome durch 1 oder 2 gleiche oder verschiedene Rest aus der Reihe
      Carboxy,
      Amino,
      (C₁-C₄)-Alkylamino,
      Hydroxy,
      (C₁-C₄)-Alkoxy,
      Halogen,
      Di-(C₁-C₄)-alkylamino,
      Carbamoyl,
      Sulfamoyl,
      (C₁-C₄)-Alkoxycarbonyl,
      (C₆-C₁₂)-Aryl und
      (C₆-C₁₂)-Aryl-(C₁-C₅)-alkyl
      ersetzt sind,
   a₂)
      (C₃-C₈)-Cycloalkyl,
      (C₆-C₁₂)-Aryl,
      (C₆-C₁₂)-Arylsulfonyl oder
      (C₃-C₉)-Heteroaryl bedeutet,
      wobei in den unter a₁) und a₂) definierten Resten jeweils (C₆-C₁₂)-Aryl bzw. (C₃-C₉)-Heteroaryl gegebenenfalls durch 1, 2 oder 3 gleiche oder verschiedene Reste aus der Reihe
      Carboxy,
      Amino,
      Nitro,
      (C₁-C₄)-Alkylamino,
      Hydroxy,
      (C₁-C₄)-Alkoxy,
      Halogen,
      Cyano,
      Di-(C₁-C₄)-alkylamino,
      Carbamoyl,
      Sulfamoyl und
      (C₁-C₄)-Alkoxycarbonyl
      substituiert ist, oder
   a₃)
      einen Rest der Formel IIa oder IIb bedeutet, 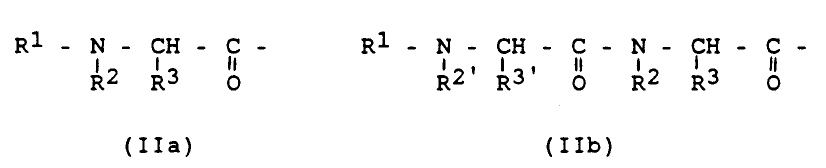
R¹
   b₁)
      Wasserstoff bedeutet oder
   b₂)
      wie A unter a₁) oder a₂) definiert ist,
   c₁)
      - R² und R²′: gleich oder verschieden sind und
      Wasserstoff oder Methyl bedeuten,
      - R³ und R^{3′}: gleich oder verschieden sind und
      Wasserstoff oder
      (C₁-C₆)-Alkyl, vorzugsweise (C₁-C₄)-Alkyl,
      das gegebenenfalls durch
      Amino,
      Benzyloxycarbonylamino,
      Hydroxy,
      Carboxy,
      Carbamoyl,
      Guanidino,
      Ureido,
      Mercapto,
      Methylmercapto,
      Phenyl,
      4-Chlorphenyl,
      4-Fluorphenyl,
      4-Nitrophenyl,
      4-Methoxyphenyl,
      4-Hydroxyphenyl,
      Phthalimido,
      4-Imidazolyl,
      3-Indolyl,
      2-Thienyl,
      3-Thienyl,
      2-Pyridyl,
      3-Pyridyl oder
      Cyclohexyl monosubstituiert ist, bedeuten,
   c₂)
      - R² und R³: und/oder R^{2′} und R^{3′} jeweils zusammen für eine [-CH₂-CH₂-CH₂-]-Kette stehen, worin eine CH₂-Gruppe durch O ersetzt sein kann, oder
   c₃)
      - R² und R³: und/oder R^{2′} und R^{3′} jeweils zusammen für 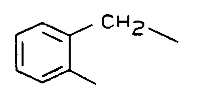 stehen,
      Cyc einem der folgenden heterocyclischen Ringe entspricht, wie 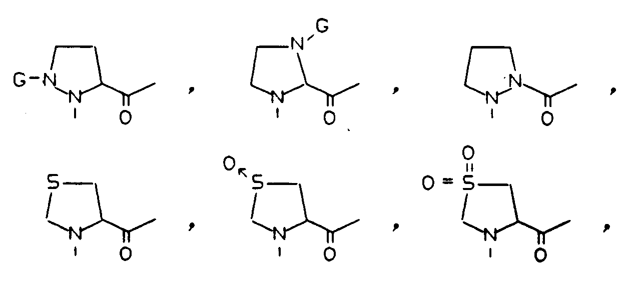 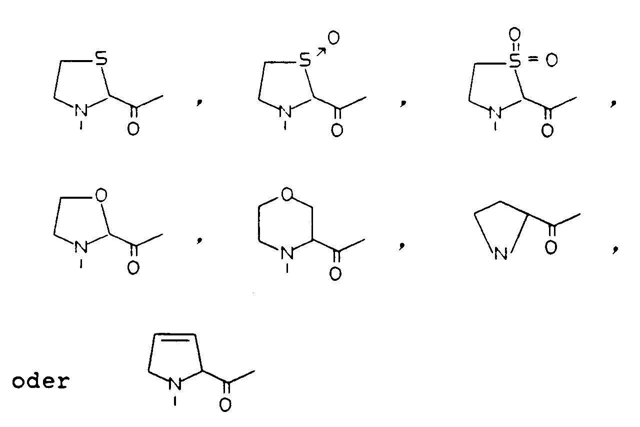 wobei G Wasserstoff bedeutet oder wie A unter a₁) oder a₂) definiert ist,
      - D: Imino
      N-Methylimino,
      Oxy oder
      Methylen bedeutet,
      - E: Carbonyl,
      C=NR⁷,
      C=N-OR⁷ oder
      Sulfinyl bedeutet oder, falls F für eine Bindung steht, auch Hydroxymethylen bedeuten kann,
      - R⁷: Wasserstoff,
      (C₁-C₈)-Alkyl oder
      (C₃-C₈)-Cycloalkyl bedeutet,
      - F: Oxy,
      Imino,
      N-Methylimino oder
      eine direkte Bindung bedeutet,
      - R⁴: (C₁-C₆)-Alkyl,
      (C₃-C₆)-Cycloalkyl,
      (C₆-C₁₂)-Aryl,
      (C₆-C₁₂)-Aryl-(C₁-C₅)-alkyl,
      (C₆-C₁₂)-Aryloxy-(C₁-C₅)-alkyl,
      (C₃-C₉)-Heteroaryl oder
      (C₃-C₉)-Heteroaryl-(C₁-C₅)-alkyl bedeutet, worin jeweils Alkyl gegebenenfalls durch 1 oder 2 gleiche oder verschiedene Reste aus der Reihe
      Carboxy,
      Amino,
      (C₁-C₄)-Alkylamino,
      Hydroxy,
      (C₁-C₄)-Alkoxy,
      Halogen,
      Di-(C₁-C₄)-alkylamino,
      Carbamoyl,
      Sulfamoyl,
      (C₁-C₄)-Alkoxycarbonyl,
      (C₆-C₁₂)-Aryl und
      (C₆-C₁₂)-Aryl-(C₁-C₅)-alkyl substituiert ist, und worin jeweils (C₆-C₁₂)-Aryl bzw. (C₃-C₉)-Heteroaryl gegebenenfalls durch 1, 2 oder 3 gleiche oder verschiedene Reste aus der Reihe
      Carboxy,
      Cyano,
      Amino,
      Nitro,
      (C₁-C₄)-Alkylamino,
      Hydroxy,
      (C₁-C₄)-Alkoxy,
      Halogen,
      Di-(C₁-C₄)-alkylamino,
      Carbamoyl,
      Sulfamoyl und
      (C₁-C₄)-Alkoxycarbonyl substituiert ist,
      und
      - R⁸: wie R³ unter c₁) definiert ist,
sowie deren physiologisch verträgliche Salze.

Unter (C₆-C₁₂)-Aryl wird beispielsweise Phenyl, Naphthyl oder Biphenylyl verstanden. Entsprechendes gilt für davon abgeleitete Reste wie Aroyl.

Alkyl und davon abgeleitete Reste wie Alkoxy können geradkettig oder verzweigt sein.

Halogen steht vorzugsweise für Fluor, Chlor oder Brom.

Ein Heteroaryl-Rest im Sinne der vorliegenden Erfindung ist der Rest eines monocyclischen oder bicyclischen (C₃-C₉)-Heteroaromaten, der im Ringsystem ein oder zwei N-Atome und/oder ein S- oder ein O-Atom enthält. Zum Begriff "Heteroaromat" siehe Garatt, Vollhardt, Aromatizität, Stuttgart 1973, Seiten 131 - 153. Beispiele geeigneter Heteroarylreste sind die Reste von Thiophen, Furan, Benzo[b]thiophen, Benzofuran, Pyrrol, Imidazol, Pyrazol, Pyridin, Pyrazin, Pyrimidin, Pyridazin, Indol, Chinolin, Isochinolin, Oxazol, Isoxazol, Thiazol, Isothiazol, Isobenzofuran, Indolizin, Isoindol, Indazol, Phthalazin, Naphthyridin, Chinoxalin, Chinazolin, Cinnolin und Furazan. Aryl, Alkyl, Heteroaryl und davon abgeleitete Reste können wie oben angegeben einfach oder, falls chemisch möglich, auch mehrfach substituiert sein.

Chiralitätszentren können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diastereomerengemische.

Als Salze kommen insbesondere Alkali- und Erdalkalisalze, Salze mit physiologisch verträglichen Aminen und Salze mit anorganischen oder organischen Säuren wie z.B. HCl, HBr, H₂SO₄, Maleinsäure, Fumarsäure in Frage.

Bevorzugt sind solche Verbindungen der Formel I, worin A gegebenenfalls substituiertes (C₁-C₈)-Alkyl, (C₁-C₈)-Alkanoyl oder gegebenenfalls substituiertes (C₁-C₈)-Alkoxycarbonyl bedeutet oder definiert ist, wie oben unter a₃), solche, worin R⁸ Wasserstoff bedeutet und solche, worin Cyc 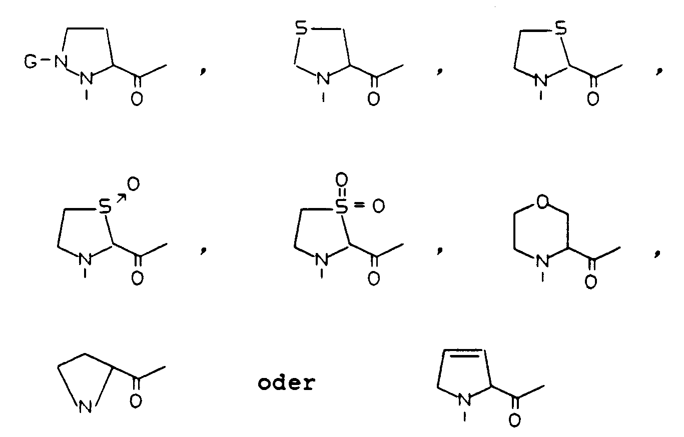
worin G für Wasserstoff steht oder wie A unter a₁) oder a₂) definiert ist, bedeutet.

Weiterhin sind Verbindungen der Formel I bevorzugt, in der D Imino, Oxy oder Methylen - besonders bevorzugt
Imino - bedeutet,
E Carbonyl bedeutet,
F Oxy oder eine direkte Bindung bedeutet und/oder
R⁴ gegebenenfalls substituiertes (C₆-C₁₂)-Aryl oder gegebenenfalls im Alkylteil und/oder gegebenenfalls im Arylteil substituiertes (C₆-C₁₂)-Aryl-(C₁-C₅)-alkyl bedeutet, wobei als Substituenten des Arylteils insbesondere Halogene, Pseudohalogene und Carboxy-Gruppen, als Substituenten des Alkylteiles insbesondere Carboxy-, (C₁-C₄)-Alkoxycarbonyl-und Aminogruppen in Frage kommen.

Besonders bevorzugt sind Verbindungen der Formel I, worin A für substituiertes (C₁-C₈)-Alkanoyl, wobei als Substituenten beispielsweise (C₆-C₁₂)-Aryl-(C₁-C₄)-alkylsulfonyl und (C₆-C₁₂)-Aryl-(C₁-C₄)-alkylsulfinyl in Frage kommen, oder für einen Rest der Formel IIa, wie oben unter a₃) definiert, steht.

Die Erfindung betrifft auch ein Verfahren zur Herstellung von Verbindungen der Formel I, das dadurch gekennzeichnet ist, daß man die Verbindungen in bekannter Weise aus den Fragmenten beispielsweise durch deren Kupplung aufbaut, gegebenenfalls eine oder mehrere temporär eingeführte Schutzgruppe(n) abspaltet, Carbonylfunktionen gegebenenfalls in die Thia-Analogen überführt und die so erhaltenen Verbindungen der Formel I gegebenenfalls in ihre physiologisch verträglichen Salze überführt.

Unter Fragmenten in obigen Sinne werden Aminosäuren, mehrere Aminosäuren enthaltende Segmente, Derivate von Aminosäuren, Derivate von Peptiden mit modifizierten Peptidbindungen ebenso wie verschiedenartig substituierte Carbonsäuren, diverse Alkohole und deren Derivate verstanden.

Die Kupplung kann dadurch erfolgen, daß man z.B. ein Fragment einer Verbindung der Formel I mit einer endständigen Carboxylgruppe oder einem reaktiven Säurederivat mit einem entsprechenden anderen Fragment, welches z.B. eine freie Aminogruppe enthält, wobei eventuell vorhandene nicht an der Reaktion beteiligte funktionelle Gruppe gegebenenfalls geschützt sein können, unter Bildung einer Amidbindung in einem inerten Lösungsmittel kondensiert. Methoden, die zur Bildung einer Amidbindung geeignet sind werden in Houben-Weyl, Methoden der organischen Chemie, Band 15/2 beschrieben.

Man führt das Verfahren vorteilhaft in der Weise durch, daß man
a) eine Verbindung der Formel IIIa mit einer Verbindung der Formel IIIb 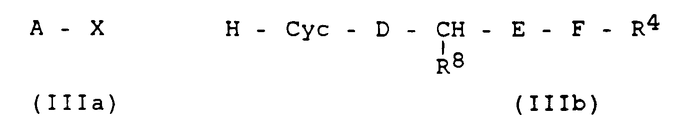 in welcher A, Cyc, D, E, F, R⁴ und R⁸ wie oben definiert sind und X für eine nucleophil ablösbare Abgangsgruppe wie OH, Cl, Br, I, Tosyl, Triflat oder, falls A ein Acylrest ist, auch für eine Aktivestergruppe steht, kondensiert,
b) eine Verbindung der Formel IVa mit einer Verbindung der Formel IVb, 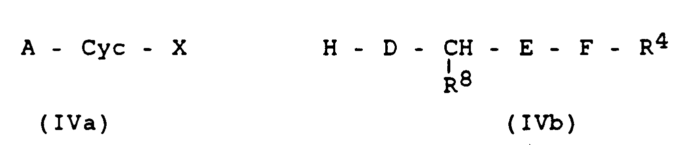 in welcher A, Cyc, E, F, R⁴ und R⁸ wie oben definiert sind, D für Imino, N-Methylimino oder Oxy steht und X für eine nucleophil ablösbare Abgangsgruppe wie OH, Cl, Br, I, Tosyl, Triflat oder, eine Aktivestergruppe steht, kondensiert oder
c) eine Verbindung der Formel Va mit einer Verbindung der Formel Vb, 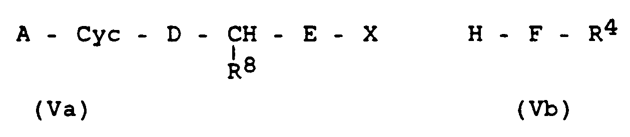 in welchen A, Cyc, D, R⁴ und R⁸ wie oben definiert sind, E mit Ausnahme von Hydroxymethylen wie oben definiert ist, F Oxy, Imino oder N-Methylimino bedeutet und X für eine nucleophil ablösbare Abgangsgruppe wie OH, Cl, Br, I, Tosyl, Triflat oder eine Aktivestergruppe steht, kondensiert.

Die Umsetzung einer Carbonsäure der Formel IIIa, IVa, bzw. Va mit der entsprechenden Verbindung der Formel IIIb, IVb bzw. Vb mit freier Aminogruppe wird vorzugsweise in einem in der Peptidchemie üblichen Lösungsmittel oder auch in Wasser/Lösungsmittel-Gemischen in Gegenwart eines geeigneten Kondensationsmittels durchgeführt, wie z.B.
1. Dicyclohexylcarbodiimid unter Zusatz von 1-Hydroxybenzotriazol (DCC/HOBt-Methode, Lit.: Chem. Ber. 103 (1970) 788)
2. Dicyclohexylcarbodiimid unter Zusatz von 3-Hydroxy-4-oxo-3,4-dihydro-1,2,3-benzotriazin (DCC/HOOBt-Methode; Lit.: Chem. Ber. 103 (1970) 2034)
3. Dicyclohexylcarbodiimid unter Zusatz von N-Hydroxysuccinimid (DCC/HONSu-Methode; Lit.: Z. Naturforsch. 21b (1966) 426)
4. Alkanphosphonsäureanhydrid, wie n-Propylphosphonsäureanhydrid (PPA-Methode; Lit.: Angew. Chemie. Int. Ed. 19 (1980) 133)
5. Dialkylphosphinsäureanhydrid, wie Methylethylphosphinsäureanhydrid (MEPA-Methode; Lit.: US-Patent 4 426 325).

Als geeignete Lösungsmittel verwendet man bei dem erfindungsgemäßen Verfahren aus Gründen der Löslichkeit meist polare Lösungsmittel wie z.B. Dimethylacetamid, Dimethylformamid, Dimethylsulfoxid, Phosphorsäure-tris-(dimethylamid), N-Methylpyrrolidon, Wasser oder Mischungen der genannten Lösungsmittel mit Wasser. Letzteres trifft vor allem beim MEPA-Verfahren zu. Aber auch Chloroform, Methylenchlorid oder Essigester kommen zum Einsatz. Die Synthese kann zwischen -10 und ca. 50°C vorzugsweise -10°C und Raumtemperatur durchgeführt werden. Vorzugsweise beginnt man bei ca. 0°C und erhöht später auf Raumtemperatur.

Die Kondensation einer Carbonsäure der Formel IVa bzw. Va mit einem Alkohol der Formel IVb bzw. Vb zu einem Carbonsäureester läßt sich vorteilhaft nach der Dicyclohexylcarbodiimid(DCC)-Methode - wie in Angew. Chem. 90, 556 (1978) beschrieben - in einem inerten Lösungsmittel wie DMF unter Katalyse von 4-Dimethylaminopyridin im Temperaturbereich zwischen -20 bis +40°C, vorzugsweise -20°C bis Raumtemperatur durchführen.

Methoden, die zur Herstellung einer modifizierten Peptidbindung geeignet sind, werden in Janssen Chimica Acta, Vol. 3, Nr. 2 [1985] beschrieben. Vorzugsweise werden die folgenden Methoden herangezogen:

Synthese von Derivaten mit reduzierten Peptidbindungen durch reduktive Aminierung von Aldehyden mit Aminosäureestern mittels Natriumcyanoborhydrid (vgl. Borch et al., J. Amer. Chem. Soc. 93 [1971] 2897; 91 [1969] 3996) oder durch N-Alkylierung von Aminen oder Aminosäurederivaten (vgl. Houben-Weyl, Methoden der Org. Chemie, Bd. 11/1), indem man
d) eine Verbindung der Formel VIa, VIb oder VIc mit einer Verbindung der Formel IIIb,

   A'-CHO (VIa)

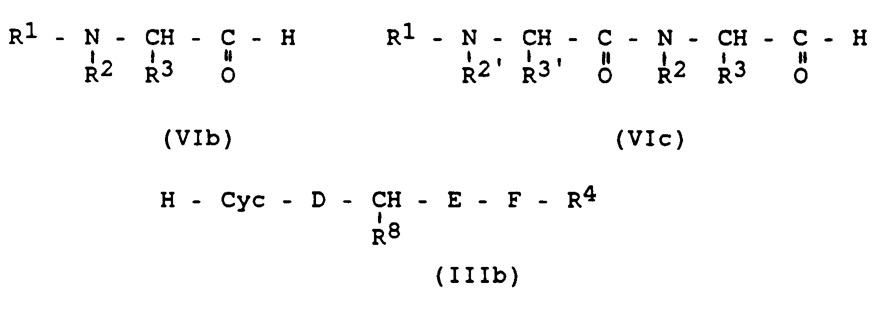

   in welchen Cyc, D, E, F, R¹, R², R^{2'}, R³, R^{3'}, R⁴ und R⁸ wie oben definiert sind und
   A' für (C₁-C₈)-Alkyl steht, das gegebenenfalls wie A unter a₁) substituiert ist, oder für (C₃-C₈)-Cycloalkyl, (C₆-C₁₂)-Aryl oder (C₃-C₉)-Heteroaryl steht, die gegebenenfalls wie A unter a₂) substituiert sind, umsetzt,
e) eine Verbindung der Formel VIIa mit einer Verbindung der Formel VIIb 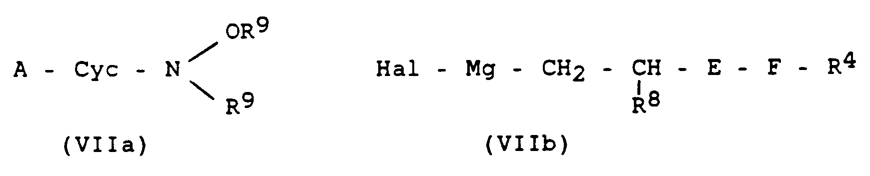 in welchen A, E, F, Cyc, R⁴ und R⁸ wie oben definiert sind, R⁹ (C₁-C₆)-Alkyl, vorzugsweise Methyl und Hal, Chlor, Brom oder Jod bedeuten, umsetzt. Man führt die Reaktion vorzugsweise in einem Ether wie Diethylether, Dibutylether, Diisoamylether oder Tetrahydrofuran bei einer Temperatur zwischen -80 °C und dem Siedepunkt des Reaktionsgemisches durch. Funktionelle Gruppen sind, falls erforderlich, temporär mit gegen metallorganische Reagenzien stabile Schutzgruppen zu schützen.

Endothiopeptide werden vorteilhaft durch Umsetzung der Verbindungen des allgemeinen Typs I mit Lawesson's Reagenz (2,4-Bis-(4-methoxyphenyl)-1,3-dithia-2,4-diphosphetan-2,4-disulfid) erhalten (vgl. Synthesis 1979, 941).

Zum temporären Schutz weiterer funktioneller Gruppen sind Schutzgruppen geeignet, wie sie in der Peptidsynthese üblicherweise verwendet werden und beispielsweise in Kontakte Merck 3/79, Seiten 14 - 22 und 1/80, Seiten 23 - 35 beschrieben sind.

Urethanschutzgruppen der Aminofunktion sind beispielsweise Pyoc, Fmoc, Fcboc, Z, Boc, Ddz, Bpoc, Z-(NO₂), Dobz, Moc, Mboc, Iboc, Adoc, Adpoc, Msc oder Pioc; bevorzugt sind Z oder Boc. Die Entfernung dieser Aminoschutzgruppen erfolgt mit Säuren, Basen oder reduktiv.

Schutzgruppen der Guanidinogruppe sind z.B. NO₂, Tosyl, Boc, Z, Mesitylen-2-sulfonyl (Mts) u.ä.. Die Abspaltung kann hydrolytisch oder hydrogenolytisch erfolgen.

Die COOH-Seitenfunktionen werden als Alkylester, vorzugsweise Methyl, Ethyl- oder tert. Butylester oder als Benzylester oder modifizierte Benzylester (p-NO₂, p-Cl, p-Br u.a.) blockiert. Die Deblockierung erfolgt durch alkalische oder saure Verseifung oder Hydrierung.

Hydroxylschutzgruppen sind beispielsweise tert. Butyl oder Benzyl.

Die erfindungsgemäßen Substanzen sind als Hemmstoffe der Prolylhydroxylase wirksam. Infolgedessen bewirken sie eine selektive Hemmung der kollagenspezifischen Hydroxylierungsreaktion, in dessen Verlauf Protein gebundenes Prolin durch das Enzym Prolylhydroxylase hydroxyliert wird. Bei Unterbindung dieser Reaktion mittels eines Inhibitors entsteht ein nichtfunktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens. Inhibitoren der Prolylhydroxylase sind deshalb geeignete Werkzeuge in der Therapie von Erkrankungen, in denen die Ablagerung von Kollagenen maßgeblich zum Krankheitsbild beiträgt. Hierzu gehören u.a. Fibrosen der Lunge, Leber und Haut (Skleroderma) sowie die Atherosklerose.

Es ist weiterhin bekannt, daß Inhibitoren der Kollagenproduktion Antitumor-Eigenschaften besitzen. Durch die Reduktion der Kollagen-Synthese und -Ablagerung wird die für das Tumorwachstum notwendige Stromaumgestaltung beeinflußt (H. Dvorak, N. Engl. J. of Med. 315 (1986) 1650); und die Inhibitoren der Baselmembranbildung sind geeignet, das Wachstum verschiedener Tumore zu unterdrücken (W. Klohs et al. J. N. C. I. 75 (1985) 353).

Außerdem ist bekannt, daß die Inhibierung der Prolylhydroxylase durch bekannte Inhibitoren wie α,α-Dipyridyl zu einer Hemmung der C1q-Biosynthese von Makrophagen führt (W. Müller et al., FEBS Lett. 90, 218f (1978)). Dadurch kommt es zu einem Ausfall des klassischen Weges der Komplementaktivierung. Inhibitoren der Prolylhydroxylase wirken daher auch als Immunsuppressiva, z.B. bei Immunkomplexkrankheiten.

Die erfindungsgemäßen Substanzen können daher als Fibrosuppressiva, Immunsuppressiva, Antiatherosklerotika und in der Tumortherapie eingesetzt werden.

Die Hemmwirkung kann in einem Enzymtest analog der Methode von B. Peterkofsky und R. DiBlasio, Anal. Biochem. 66, 279 - 286 (1975) bestimmt werden. Dabei wird unterhydroxyliertes Kollagen mit Prolylhydroxylase in Gegenwert von Eisen(II)ionen, α-Ketoglutarat und Ascorbat enzymatisch hydroxyliert.

Zur Bestimmung eines irreversiblen Hemmtyps wird das Enzym in Gegenwert von Eisen(II)ionen, α-Ketoglutarat und Ascorbat mit den Hemmstoffen für unterschiedliche Zeiten vorinkubiert und anschließend in Gegenwart von Peptidsubstrat die Restaktivität des Enzyms bestimmt. Zur Aktivitätsbestimmung können sowohl die oben beschriebene Methode nach Peterkofsky und DiBlasio als auch andere Methoden wie bei K.I. Kivirikko und R. Myllylä, Meth. Enzym. 82, 245 - 304 (1982) beschrieben, eingesetzt werden.

Die Hemmwirkung kann auch in der Zell- oder Gewebekultur bestimmt werden. Dazu können Fibroblasten oder andere Kollagen produzierende Zellen bzw. Calvarien oder andere Kollagen produzierende Organe eingesetzt werden. Die Wirkung der Substanzen kann über die Absenkung des Hydroxyprolin/Prolin-Quotienten bei Verfütterung von ¹⁴C-Prolin bestimmt werden.

Die antifibrotische Wirkung kann im Modell der Tetrachlorkohlenstoff-induzierten Leberfibrose bestimmt werden. Dazu werden Ratten mit CCl₄ (1 ml/kg) gelöst in Olivenöl zweimal wöchentlich behandelt. Die Prüfsubstanz wird täglich, gegebenenfalls zweimal täglich per os oder intraperitoneal - gelöst in einem geeigneten verträglichen Lösungsmittel - verabreicht. Das Ausmaß der Leberfibrose wird histologisch bestimmt; der Anteil Kollagen in der Leber per Hydroxyprolinbestimmung - wie bei Kivirikko et al. (Anal. Biochem. 19, 249 f, (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentrationen von 1 - 100 mg/kg wirksam. Ein anderes Modell zur Evaluierung der antifibrotischen Wirkung ist das der Bleomycin-induzierten Lungenfibrose wie bei Kelley et al. (J. Lab. Clin. Med. 96, 954, (1980)) beschrieben. Für die Evaluierung der Wirkung der erfindungsgemäßen Verbindungen auf Granulationsgewebe kann das Modell des Wattebauschgranuloms, wie bei Meier et al., Experientia 6, 469 (1950) beschrieben, herangezogen werden.

Die Erfindung betrifft daher die Verwendung einer Verbindung der Formel I als Inhibitor der Prolylhydroxylase sowie deren Verwendung als Heilmittel bei Säugern und beim Menschen, insbesondere als Fibrosuppressiva, Immunsuppressiva, Antiatherosklerosika und in der Tumortherapie.

Die Erfindung betrifft weiterhin pharmazeutische Zubereitungen, die eine wirksame Menge einer Verbindung der Formel I und einen physiologisch unbedenklichen Träger enthalten sowie ein Verfahren zur Herstellung dieser Zubereitungen, das dadurch gekennzeichnet ist, daß man den Wirkstoff zusammen mit dem Träger und gegebenenfalls weiteren Hilfs- oder Zusatzstoffen in eine geeignete Darreichungsform bringt. Der Wirkstoffanteil in diesen Zubereitungen beträgt in der Regel 0,1 bis 96 %.

Die Anwendung kann intranasal, buccal, intravenös, subcutan oder peroral erfolgen. Die Dosierung des Wirkstoffes hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragées, Steckkapseln, wäßrige, alkoholische oder ölige Suspensionen oder wäßrige, alkoholische oder ölige Lösungen. Als inerte Träger kommen z.B. Gummi arabicum, Magnesiumcarbonat, Kaliumphosphat, Milchzucker, Glucose oder Stärke, insbesondere Maisstärke in Betracht. Dabei kann die Zubereitung sowohl als Trocken- als auch als Feuchtgranulat erfolgen. Als ölige Trägerstoffe oder Lösungsmittel kommen beispielsweise pflanzliche oder tierische Öle in Betracht, wie Sonnenblumenöl oder Lebertran.

Zur subcutanen oder intravenösen Applikation werden die aktiven Verbindungen oder deren physiologisch verträgliche Salze, gewünschtenfalls mit den dafür üblichen Substanzen wie Lösungsvermittler oder weiteren Hilfsstoffen in Lösung, Suspension oder Emulsion gebracht. Dazu kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die folgenden Beispiele dienen zur Illustration der vorliegenden Erfindung, ohne daß diese darauf beschränkt wäre.

Verzeichnis der verwendeten Abkürzungen:
- AA: Aminosäureanalyse
- Ac: Acetyl
- Boc: tert.-Butoxycarbonyl
- DC: Dünnschichtchromatographie
- DCC: Dicyclohexylcarbodiimid
- DCH: Dicyclohexylharnstoff
- DMF: Dimethylformamid
- DMSO: Dimethylsulfoxid
- EE: Essigsäureethylester
- FAB: Fast atom bombardment
- Fmoc: 9-Fluorenylmethyloxycarbonyl
- HOBt: 1-Hydroxybenzotriazol
- HOSu: 1-Hydroxysuccinimid
- HPhe: Homophenylalanin
- i.V.: im Vakuum
- M: Molekularpeak
- MeOH: Methanol
- MS: Massenspektrum
- CHN: Elementaranalyse
- NEM: N-Ethylmorpholin
- tBu: tert. Butyl
- Pht: Phthalyl
- Schmp.: Schmelzpunkt
- THF: Tetrahydrofuran
- Z: Benzyloxycarbonyl
- h: Stunde
- min: Minute
- RT: Raumtemperatur
- Tic: 1,2,3,4-Tetrahydroisochinolin
- Phg: Phenylglycin
Die sonstigen für Aminosäuren und Schutzgruppen verwendeten Abkürzungen entsprechen dem in der Peptidchemie üblichen Buchstaben-Code, wie er z.B. in Europ. J. Biochem. 138, 9 - 37 (1984) beschrieben ist. Falls nicht ausdrücklich anders angegeben, handelt es sich immer um Aminosäuren der L-Konfiguration.

### Beispiele

### A.1.

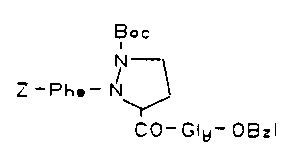

### A.1.1. 1-tert.-Butoxycarbonylpyrazolidincarbonsäureethylester

53,5 g Diazald® in 750 ml tert.-Butylmethylether werden auf 0 °C gekühlt und mit 16,5 KOH, gelöst in 250 ml Ethanol/Wasser (9:1), versetzt. Man erwärmt auf Raumtemperatur und rührt 1 h bei Raumtemperatur. 25,0 g (0,25 M) Acrylsäureethylester in 20 ml tert.-Butylmethylether werden über 1,5 h zugetropft, wobei zum Ende hin 40 ml Wasser addiert wird.
Zur Aufarbeitung werden 800 ml Diethylether zugefügt, die organische Phase abgetrennt, mit gesättigter NaCl-Lösung gewaschen und über Na₂SO₄ getrocknet. Der Rückstand wird in 500 ml DMF gelöst, mit 55 ml Triethylamin, 100 mg N,N-Dimethylaminopyridin und 54 g (0,25 M) Pyrokohlensäure-di-tert.-butylester versetzt.
Nach Chromatographie an Kieselgel werden 8 g Δ₂-1-tert.-Butoxycarbonylpyrazolin-3-carbonsäureethylester erhalten. 6 g des Pyrazolinderivates werden in 100 ml Essigsäure gelöst und mit 7 g Zink-Pulver versetzt und gerührt.
Die Reaktionslösung wird nach 2 h abfiltriert, das Filtrat im Vakuum einrotiert und der Rückstand über Kieselgel chromatographiert. Man erhält 4,5 g 1-tert.-Butoxycarbonylpyrazolidin-3-carbonsäureethylester.
¹H-NMR: δ (TMS) = 1,3; 1,5; 2,0-2,5; 3,4-3,7; 3,9; 4.25 ppm.
MS (FAB) = 245 (M + H)

### A.1.2.

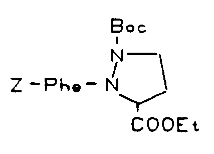

598 mg Z-Phe-OH in 5 ml CH₂Cl₂ werden 0 °C gekühlt und mit einer Lösung von 420 mg DCC in 1 ml CH₂Cl₂ versetzt, 30 Minuten bei 0 °C, dann 30 Minuten bei Raumtemperatur gerührt. Nach Abfiltrieren des Harnstoffes wird im Vakuum eingedampft und der Rückstand über Kieselgel chromatographiert. Man erhält 610 mg der Zielverbindung.
¹H-NMR: δ (TMS) = 1,35 und 1,5 (^{t}Bu), 4,2 (OCH₂) ppm
MS (FAB) = 526 (M + H)

### A.1.3.

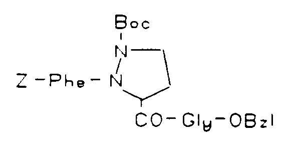

600 mg des Dipeptids werden mit 4 n NaOH in Dioxan/Methanol bei Titration gegen Thymolphthalin verseift. Man stellt mit 1 n HCl anschließend auf pH 2 ein, extrahiert mit EE, wäscht die organische Phase mit gesättigter NaCl-Lösung und trocknet über Na₂SO₄.
Der Rückstand (550 mg) wird mit 343 mg H-Gly-OBzl .
Toluolsulfonsäure in 3 ml CH₂Cl₂ gelöst, auf 0 °C gekühlt und mit einer Lösung von 230 mg DCC in CH₂Cl₂ bei Zugabe von 150 µl NEM versetzt. Man rührt 15 Minuten bei 0 °C, 20 Minuten bei Raumtemperatur und rotiert das Lösungsmittel im Vakuum ein. Durch Chromatographie an Kieselgel (Essigester/Petrolether 2:1) lassen sich die Diastereomeren trennen. Man erhält 150 mg der R-konfigurierten sowie 130 mg des S-konfigurierten Pyrazolidinderivates.
¹H-NMR: δ (TMS) = 1,35 ((R)-^{t}Bu), 1,5 ((S)-^{t}Bu) ppm.
MS (FAB) = 645 (M + H)

### A.2.

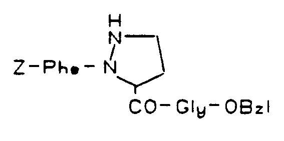

120 mg des Boc-geschützten Pyrazolidinderivates aus A.1.3. in 4 ml 9 n etherischer HCl-Lösung werden 45 Minuten bei Raumtemperatur gerührt. Es wird anschließend im Vakuum einrotiert, erneut mit Diethylether aufgenommen und im Vakuum einrotiert. Man erhält 100 mg der Zielverbindung als Hydrochlorid.
MS (FAB) = 545 (M + H)

### B.1.

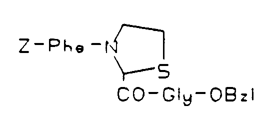

### B.1.1. Thiazolidin-2-carbonsäurethylester

22,6 g Mercaptoethylamin Hydrochlorid und 20,4 g Glyoxylsäureethylester werden mit 100 g Molekularsieb 3A 45 min. auf dem Wasserbad erwärmt. Es wird abfiltriert, im Vakuum eingedampft, der Rückstand über Kieselgel filtriert und aus Methylenchlorid/tert.-Butylmethylether kristallisiert. Man erhält 27 g des Thiazolidin-Derivates.

### B.1.2.

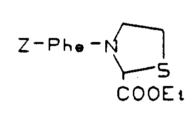

1,98 g Thiazolidin-2-carbonsäureethylester, 2,99 g Z-Phe-OH und 1,35 g HOBt werden in wenig DMF gelöst, auf 0 °C gekühlt und nacheinander mit 2,1 g DCC und 1,28 ml NEM versetzt. Man rührt 1 h bei 0 ° und weitere 10 h bei RT. Nach Abfiltrieren des Harnstoffs wird i.V. einrotiert, der Rückstand in Essigester aufgenommen und über Kieselgel filtriert. Man erhält 4 g der Zielverbindung als Öl, das direkt nach B.1.3. weiterverarbeitet wird.

### B.1.3.

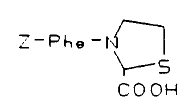

4 g des Reaktionsprodukts aus B.1.2. werden in 20 ml Dioxan und 5 ml H₂O gelöst und bei Titration gegen Thymolphthalein mit 1 N NaOH-Lösung verseift. Nach vollständigem Umsatz bei DC-Kontrolle wird mit KHSO₄-Lösung angesäuert und die wäßrige Phase mit EE extrahiert. die organische Phase wird mit gesättigter NaCl-Lösung gewaschen und über Na₂SO₄ getrocknet. Nach Einrotieren i.V. erhält man 3,6 g der Zielverbindung als Öl.
MS (FAB) = 415 (M + H)

### B.1.4.

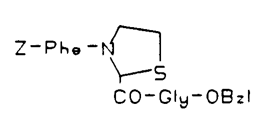

3,4 g des Reaktionsproduktes aus B.1.3., 2,7 g des Toluolsulfonsäuresalzes von H-Gly-OBzl und 1,1 g HOBt werden in 8 ml DMF gelöst, auf 0 °C gekühlt und mit 1,7 g DCC und 1,1 ml NEM versetzt. Man rührt 1 h bei 0 °C und weitere 10 h bei RT. Es wird vom Harnstoff abfiltriert, i.V. einrotiert, der Rückstand in EE aufgenommen, mit KHSO₄-Lösung, mit KHCO₃-Lösung sowie mit NaCl-Lösung gewaschen, über Na₂SO₄ getrocknet und das Lösungsmittel i.V. einrotiert. Das Rohprodukt wird durch Chromatographie an Kieselgel (Diethylether) gereinigt. Man erhält 4 g der Zielverbindung.
¹H-NMR: δ (TMS) = 2,8-3,2; 3,6-5,6; 7,0-7,6 ppm
MS (FAB) = 562 (M + H)

### B.2.

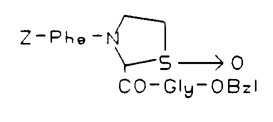

1,4 g des Tripeptids aus B.1.4. werden in 5 ml Methanol gelöst und mit 0,35 ml 30 %iger H₂O₂-Lösung versetzt. Man rührt 20 h bei RT, engt i.V. bis fast zur Trockne ein und chromatographiert den Rückstand an Kieselgel (CH₂Cl₂/CH₃OH : 25/2). Man isoliert 150 mg (I) und 200 mg (II) der Sulfoxide als Diastereomere.
- I:: ¹H-NMR: δ (TMS) = 2,9-3,2; 3,5; 3,85; 4,1; 4,35; 4,6; 5,0-5,2; 5,45; 7,1-7,5 ppm
MS (FAB) = 578 (M + H)
- II:: ¹H-NMR: δ (TMS) = 2,8-3,2; 7,1-7,4 ppm.
MS (FAB) = 578 (M + H)

### C.1.

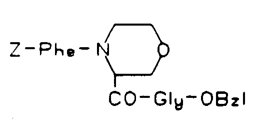

### C.1.1. Morpholin-2-carbonsäureethylester

Synthese nach Y. Kogami, K. Okawa, Pept. Chemistry 1985, 153-156.

### C.1.2.

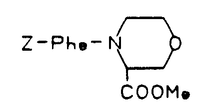

533 mg Morpholin-2-carbonsäuremethylester und 1090 mg Z-Phe-OH werden zusammen in 10 ml CH₂Cl₂ gelöst, die Lösung auf 0 °C gekühlt und mit 760 mg DCC und 0,45 ml NEM versetzt. Man rührt 5 h bei RT, filtriert vom Harnstoff ab und engt das Lösungsmittel i.V. ein. Der Rückstand wird in EE aufgenommen und durch Chromatographie an Kieselgel (Diethylether) gereinigt. Man erhält 600 mg der Zielverbindung.
MS (FAB) = 427 (M + H)
¹H-NMR: δ (TMS) = 3,65 und 3,8 (OCH₃) ppm.

### C.1.3.

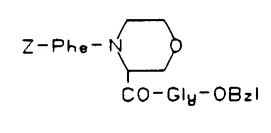

600 mg des Reaktionsproduktes aus C.1.2. werden nach den Bedingungen unter B.1.3. verseift. Man erhält 430 mg der Säure, die mit 352 mg des p-Toluolsulfonsäuresalzes von H-Gly-OBzl in 5 ml DMF und 5 ml CH₂Cl₂ gelöst wird. Man kühlt auf 0 °C und addiert 215 mg DCC und 0,13 ml NEM. Man erwärmt auf RT und rührt 2 h. Nach Abfiltrieren von DCH und Einengen des Lösungsmittels im Vakuum wird der Rückstand durch Chromatographie an Kieselgel (EE/n-Hexan : 2/1) gereinigt. Man erhält 400 mg der Zielverbindung.
¹H-NMR: δ (TMS) = 2,1; 3,0-3,2; 3,4; 3,65; 4,9-5,2; 5,45; 7,1-7,5 ppm.
MS (FAB) = 560 (M + H)

### D.1.

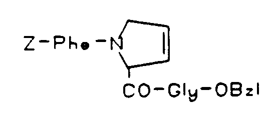

### D.1.1.

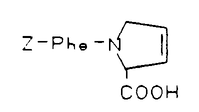

50 mg L-3,4-Dihydroprolin werden in 0,44 ml 1 N NaOH gelöst und mit 175 mg Z-Phe-OSu in 2 ml Ethanol versetzt. Bei kontinuierlichem Einstellen auf pH = 9 wird 2 h bei RT gerührt. Mit wäßriger HCl-Lösung wird anschließend auf pH = 2 eingestellt und mit EE extrahiert. Durch Trocknen der organischen Phase mit Na₂SO₄ und Einengen des Lösungsmittels i.V. wird die Zielverbindung als Rohprodukt erhalten und direkt nach D.1.2. weiter umgesetzt.

### D.1.2.

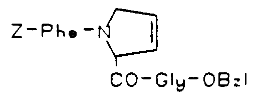

Das Rohprodukt aus D.1.1. wird mit 150 mg des p-Toluolsulfonsäuresalzes von H-Gly-OBzl und 60 mg HOBt in 5 ml CH₂Cl₂ gelöst, die Lösung auf 0 °C gekühlt und mit 91 mg DCC und 56 µl NEM versetzt. Man rührt 1 h bei 0 °C und 2 h bei RT. Nach Abfiltrieren des DCH's wird das Lösungsmittel i.V. eingeengt und der Rückstand an Kieselgel chromatographiert. Man erhält 40 mg der Zielverbindung.
¹H-NMR: δ (TMS) = 3,05 (CH₂-Phe); 5,1 und 5,2 (CH₂-OCO) ppm.
MS (FAB) = 542 (M + H)

### E.1.

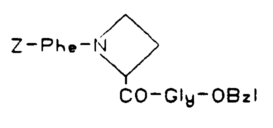

### E.1.1.

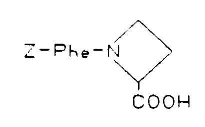

500 mg L-Azetidin-2-carbonsäure werden in 4,9 ml 1 N NaOH gelöst. Man addiert 4 ml Ethanol und 1,96 g Z-Phe-OSu und rührt 2 h bei RT. Mit KHSO₄-Lösung wird anschließend auf pH = 2 eingestellt und mehrfach mit EE extrahiert. Nach Trocknen über Na₂SO₄ wird der Rückstand nach E.1.2. weiter umgesetzt.

### E.1.2.

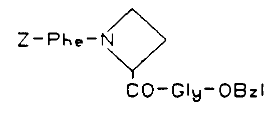

Das Rohprodukt aus E.1.1. wird mit 1,68 g der p-Toluolsulfonsäuresalzes von H-Gly-OBzl, 675 mg HOBt, 1,03 g DCC und 0,64 ml NEM in 15 ml CH₂Cl₂ gelöst und 4 h bei RT gerührt. Abfiltrieren vom Harnstoff, Einengen im Vakuum und Kristallisation des Rückstands aus Diethylether liefert 1,8 g des Tripeptids.
NMR: 2,3-2,5; 2,8-3,0; 3,3; 4,1; 4,4; 4,85; 5,1; 5,2; 5,5; 7,1-7,5 ppm
MS (FAB): 530 (M + H)

## Patentansprüche (Patentansprüche für folgende(n) Vertragsstaat(en): AT, BE, CH, DE, FR, GB, IT, LI, LU, NL, SE)

1. Verbindungen der Formel I 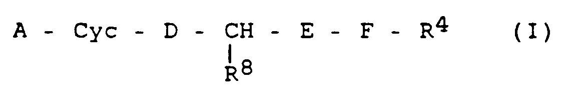 in welcher
A
a₁)
(C₁-C₈)-Alkyl,
(C₁-C₈)-Alkanoyl,
(C₁-C₈)-Alkoxycarbonyl oder
(C₁-C₈)-Alkylsulfonyl bedeutet,
in welchen jeweils 1, 2 oder 3 Wasserstoffatome gegebenenfalls durch 1, 2 oder 3 gleiche oder verschiedene Reste aus der Reihe
Carboxy,
Amino,
(C₁-C₄)-Alkylamino,
Hydroxy,
(C₁-C₄)-Alkoxy,
Halogen,
Di-(C₁-C₄)-alkylamino,
Carbamoyl,
Sulfamoyl,
(C₁-C₄)-Alkoxycarbonyl,
(C₆-C₁₂)-Aryl und
(C₆-C₁₂)-Aryl-(C₁-C₅)-alkyl ersetzt sind, oder in welchen jeweils 1 Wasserstoffatom gegebenenfalls durch einen Rest aus der Reihe
(C₃-C₈)-Cycloalkyl,
(C₁-C₄)-Alkylsulfonyl,
(C₁-C₄)-Alkylsulfinyl,
(C₆-C₁₂)-Aryl-(C₁-C₄)-alkylsulfonyl,
(C₆-C₁₂)-Aryl-(C₁-C₄)-alkylsulfinyl,
(C₆-C₁₂)-Aryloxy,
(C₃-C₉)-Heteroaryl und
(C₃-C₉)-Heteroaryloxy
und
1 oder 2 Wasserstoffatome durch 1 oder 2 gleiche oder verschiedene Reste aus der Reihe
Carboxy,
Amino,
(C₁-C₄)-Alkylamino,
Hydroxy,
(C₁-C₄)-Alkoxy,
Halogen,
Di-(C₁-C₄)-alkylamino,
Carbamoyl,
Sulfamoyl,
(C₁-C₄)-Alkoxycarbonyl,
(C₆-C₁₂)-Aryl und
(C₆-C₁₂)-Aryl-(C₁-C₅)-alkyl
ersetzt sind,
a₂)
(C₃-C₈)-Cycloalkyl,
(C₆-C₁₂)-Aryl,
(C₆-C₁₂)-Arylsulfonyl oder
(C₃-C₉)-Heteroaryl bedeutet,
wobei in den unter a₁) und a₂) definierten Resten jeweils (C₆-C₁₂)-Aryl bzw. (C₃-C₉)-Heteroaryl gegebenenfalls durch 1, 2 oder 3 gleiche oder verschiedene Reste aus der Reihe
Carboxy,
Amino,
Nitro,
(C₁-C₄)-Alkylamino,
Hydroxy,
(C₁-C₄)-Alkoxy,
Halogen,
Cyano,
Di-(C₁-C₄)-alkylamino,
Carbamoyl,
Sulfamoyl und
(C₁-C₄)-Alkoxycarbonyl
substituiert ist, oder
a₃)
einen Rest der Formel IIa oder IIb bedeutet, 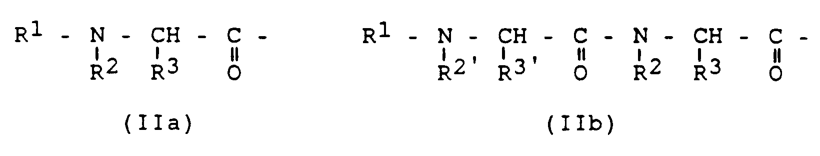
R¹
b₁)
Wasserstoff bedeutet oder
b₂
wie A unter a₁) oder a₂) definiert ist,
c₁)
R² und R²′ gleich oder verschieden sind und Wasserstoff oder Methyl bedeuten,
R³ und R^{3′} gleich oder verschieden sind und
Wasserstoff oder
(C₁-C₆)-Alkyl, vorzugsweise (C₁-C₄)-Alkyl,
das gegebenenfalls durch
Amino,
Benzyloxycarbonylamino,
Hydroxy,
Carboxy,
Carbamoyl,
Guanidino,
Ureido,
Mercapto,
Methylmercapto,
Phenyl,
4-Chlorphenyl,
4-Fluorphenyl,
4-Nitrophenyl,
4-Methoxyphenyl,
4-Hydroxyphenyl,
Phthalimido,
4-Imidazolyl,
3-Indolyl,
2-Thienyl,
3-Thienyl,
2-Pyridyl,
3-Pyridyl oder
Cyclohexyl monosubstituiert ist, bedeuten,
c₂)
R² und R³ und/oder R^{2′} und R^{3′} jeweils zusammen für eine [-CH₂-CH₂-CH₂-]-Kette stehen, worin eine CH₂-Gruppe durch O ersetzt sein kann, oder
c₃)
R² und R³ und/oder R^{2′} und R^{3′} jeweils zusammen für 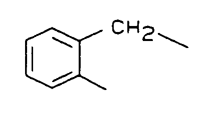 stehen,
Cyc einem der folgenden heterocyclischen Ringe entspricht, wie 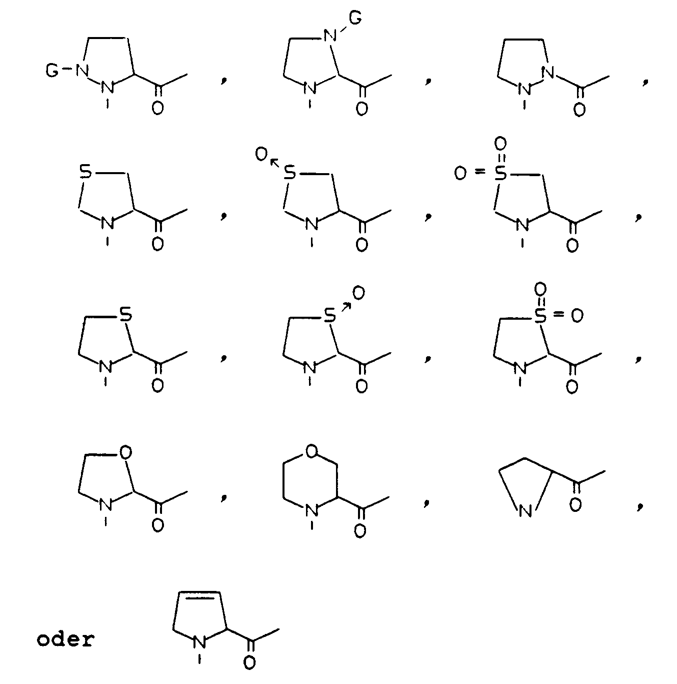 wobei G Wasserstoff bedeutet oder wie A unter a₁) oder a₂) definiert ist,
D Imino N-Methylimino,
Oxy oder
Methylen bedeutet,
E Carbonyl,
C=NR⁷,
C=N-OR⁷ oder
Sulfinyl bedeutet oder, falls F für eine Bindung steht, auch Hydroxymethylen bedeuten kann,
R⁷ Wasserstoff,
(C₁-C₈)-Alkyl oder
(C₃-C₈)-Cycloalkyl bedeutet,
F Oxy,
Imino,
N-Methylimino oder eine direkte Bindung bedeutet,
R⁴ (C₁-C₆)-Alkyl,
(C₃-C₆)-Cycloalkyl,
(C₆-C₁₂)-Aryl,
(C₆-C₁₂)-Aryl-(C₁-C₅)-alkyl,
(C₆-C₁₂)-Aryloxy-(C₁-C₅)-alkyl,
(C₃-C₉)-Heteroaryl oder
(C₃-C₉)-Heteroaryl-(C₁-C₅)-alkyl bedeutet, worin jeweils Alkyl gegebenenfalls durch 1 oder 2 gleiche oder verschiedene Reste aus der Reihe
Carboxy,
Amino,
(C₁-C₄)-Alkylamino,
Hydroxy,
(C₁-C₄)-Alkoxy,
Halogen,
Di-(C₁-C₄)-alkylamino,
Carbamoyl,
Sulfamoyl,
(C₁-C₄)-Alkoxycarbonyl,
(C₆-C₁₂)-Aryl und
(C₆-C₁₂)-Aryl-(C₁-C₅)-alkyl substituiert ist, und worin jeweils (C₆-C₁₂)-Aryl bzw. (C₃-C₉)-Heteroaryl gegebenenfalls durch 1, 2 oder 3 gleiche oder verschiedene Reste aus der Reihe
Carboxy,
Cyano,
Amino,
Nitro,
(C₁-C₄)-Alkylamino,
Hydroxy,
(C₁-C₄)-Alkoxy,
Halogen,
Di-(C₁-C₄)-alkylamino,
Carbamoyl,
Sulfamoyl und
(C₁-C₄)-Alkoxycarbonyl substituiert ist,
und
R⁸ wie R³ unter c₁) definiert ist,
sowie deren physiologisch verträgliche Salze.

2. Verbindung der Formel I gemäß Anspruch 1, in welcher A gegebenenfalls substituiertes (C₁-C₈)-Alkyl, (C₁-C₈)-Alkanoyl oder gegebenenfalls substituiertes (C₁-C₈)-Alkoxycarbonyl bedeutet oder definiert ist, wie in Anspruch 1 unter a₃) sowie deren physiologisch verträgliche Salze.

3. Verbindung der Formel I gemäß einem oder mehreren der Ansprüche 1 bis 2, in welcher R⁸ Wasserstoff bedeutet, sowie deren physiologisch verträgliche Salze.

4. Verbindung der Formel I gemäß einem oder mehreren der Ansprüche 1 bis 3, in welcher
Cyc 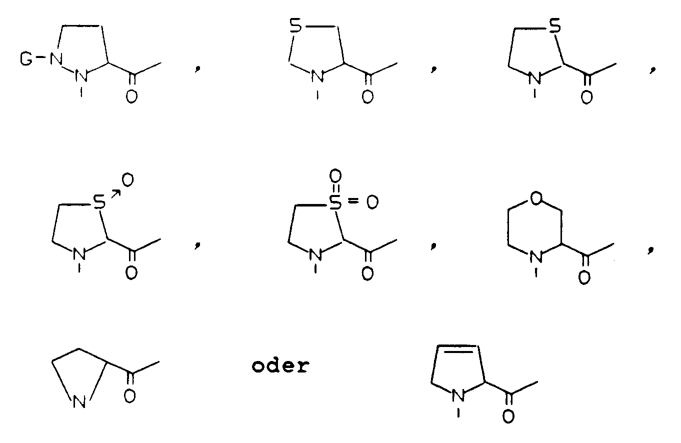 worin G für Wasserstoff steht oder wie A unter a₁) oder a₂) in Anspruch 1 definiert ist, bedeutet, sowie deren physiologisch verträgliche Salze.

5. Verbindung der Formel I gemäß einem oder mehreren der Ansprüche 1 bis 4, in welcher D Imino, Oxy oder Methylen bedeutet, sowie deren physiologisch verträgliche Salze.

6. Verbindung der Formel I gemäß einem oder mehreren der Ansprüche 1 bis 5, in welcher E Carbonyl bedeutet, sowie deren physiologisch verträgliche Salze.

7. Verbindung der Formel I gemäß einem oder mehreren der Ansprüche 1 bis 6, in welcher F Oxy oder eine direkte Bindung bedeutet, sowie deren physiologisch verträgliche Salze.

8. Verbindung der Formel I gemäß einem oder mehreren der Ansprüche 1 bis 7, in welcher
R⁴ gegebenenfalls substituiertes (C₆-C₁₂)-Aryl oder gegebenenfalls in Alkylteil und/oder gegebenenfalls im Arylteil substituiertes (C₆-C₁₂)-Aryl-(C₁-C₅)-alkyl bedeutet, sowie deren physiologisch verträgliche Salze.

9. Verfahren zur Herstellung einer Verbindung der Formel I, das dadurch gekennzeichnet ist, daß man die Verbindung in bekannter Weise aus den Fragmenten beispielsweise durch deren Kupplung aufbaut, gegebenenfalls eine oder mehrere temporär eingeführte Schutzgruppe(n) abspaltet, Carbonylfunktionen gegebenenfalls in die Thia-Analogen überführt und die so erhaltene Verbindung der Formel I gegebenenfalls in ihr physiologisch verträgliches Salz überführt.

10. Verwendung einer Verbindung der Formel I gemäß einem oder mehreren der Ansprüche 1 bis 8 als Heilmittel.

11. Verwendung einer Verbindung der Formel I gemäß einem oder mehreren der Ansprüche 1 bis 8 als Inhibitor der Prolylhydroxylase.

12. Pharmazeutische Zubereitung enthaltend eine Verbindung der Formel I gemäß einem oder mehreren der Ansprüche 1 bis 8 und einen physiologisch unbedenklichen Träger.

## Patentansprüche (Patentansprüche für folgende(n) Vertragsstaat(en): ES, GR)

1. Verfahren zur Herstellung einer Verbindungen der Formel I 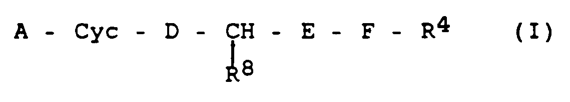 in welcher
A
a₁)
(C₁-C₈)-Alkyl,
(C₁-C₈)-Alkanoyl,
(C₁-C₈)-Alkoxycarbonyl oder
(C₁-C₈)-Alkylsulfonyl bedeutet,
in welchen jeweils 1, 2 oder 3 Wasserstoffatome gegebenenfalls durch 1, 2 oder 3 gleiche oder verschiedene Reste aus der Reihe
Carboxy,
Amino,
(C₁-C₄)-Alkylamino,
Hydroxy,
(C₁-C₄)-Alkoxy,
Halogen,
Di-(C₁-C₄)-alkylamino,
Carbamoyl,
Sulfamoyl,
(C₁-C₄)-Alkoxycarbonyl,
(C₆-C₁₂)-Aryl und
(C₆-C₁₂)-Aryl-(C₁-C₅)-alkyl ersetzt sind, oder in welchen jeweils 1 Wasserstoffatom gegebenenfalls durch einen Rest aus der Reihe
(C₃-C₈)-Cycloalkyl,
(C₁-C₄)-Alkylsulfonyl,
(C₁-C₄)-Alkylsulfinyl,
(C₆-C₁₂)-Aryl-(C₁-C₄)-alkylsulfonyl,
(C₆-C₁₂)-Aryl-(C₁-C₄)-alkylsulfinyl,
(C₆-C₁₂)-Aryloxy,
(C₃-C₉)-Heteroaryl und
(C₃-C₉)-Heteroaryloxy
und
1 oder 2 Wasserstoffatome durch 1 oder 2 gleiche oder verschiedene Reste aus der Reihe
Carboxy,
Amino,
(C₁-C₄)-Alkylamino,
Hydroxy,
(C₁-C₄)-Alkoxy,
Halogen,
Di-(C₁-C₄)-alkylamino,
Carbamoyl,
Sulfamoyl,
(C₁-C₄)-Alkoxycarbonyl,
(C₆-C₁₂)-Aryl und
(C₆-C₁₂)-Aryl-(C₁-C₅)-alkyl
ersetzt sind,
a₂
(C₃-C₈)-Cycloalkyl,
(C₆-C₁₂)-Aryl,
(C₆-C₁₂)-Arylsulfonyl oder
(C₃-C₉)-Heteroaryl bedeutet,
wobei in den unter a₁) und a₂) definierten Resten jeweils (C₆-C₁₂)-Aryl bzw. (C₃-C₉)-Heteroaryl gegebenenfalls durch 1, 2 oder 3 gleiche oder verschiedene Reste aus der Reihe
Carboxy,
Amino,
Nitro,
(C₁-C₄)-Alkylamino,
Hydroxy,
(C₁-C₄)-Alkoxy,
Halogen,
Cyano,
Di-(C₁-C₄)-alkylamino,
Carbamoyl,
Sulfamoyl und
(C₁-C₄)-Alkoxycarbonyl
substituiert ist, oder
a₃) einen Rest der Formel IIa oder IIb bedeutet, 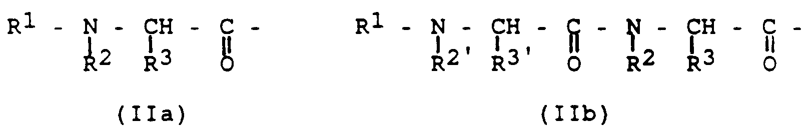
R¹
b₁)
Wasserstoff bedeutet oder
b₂)
wie A unter a₁) oder a₂) definiert ist,
c₁)
R² und R²′ gleich oder verschieden sind und
Wasserstoff oder Methyl bedeuten,
R³ und R^{3′} gleich oder verschieden sind und
Wasserstoff oder
(C₁-C₆)-Alkyl, vorzugsweise (C₁-C₄)-Alkyl,
das gegebenenfalls durch
Amino,
Benzyloxycarbonylamino,
Hydroxy,
Carboxy,
Carbamoyl,
Guanidino,
Ureido,
Mercapto,
Methylmercapto,
Phenyl,
4-Chlorphenyl,
4-Fluorphenyl,
4-Nitrophenyl,
4-Methoxyphenyl,
4-Hydroxyphenyl,
Phthalimido,
4-Imidazolyl,
3-Indolyl,
2-Thienyl,
3-Thienyl,
2-Pyridyl,
3-Pyridyl oder
Cyclohexyl monosubstituiert ist, bedeuten,
c₂)
R² und R³ und/oder R^{2′} und R^{3′} jeweils zusammen für eine [-CH₂-CH₂-CH₂-]-Kette stehen, worin eine CH₂-Gruppe durch O ersetzt sein kann, oder
c₃)
R² und R³ und/oder R^{2′} und R^{3′} jeweils zusammen für 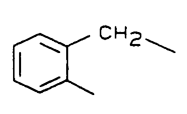 stehen,
Cyc einem der folgenden heterocyclischen Ringe entspricht, wie 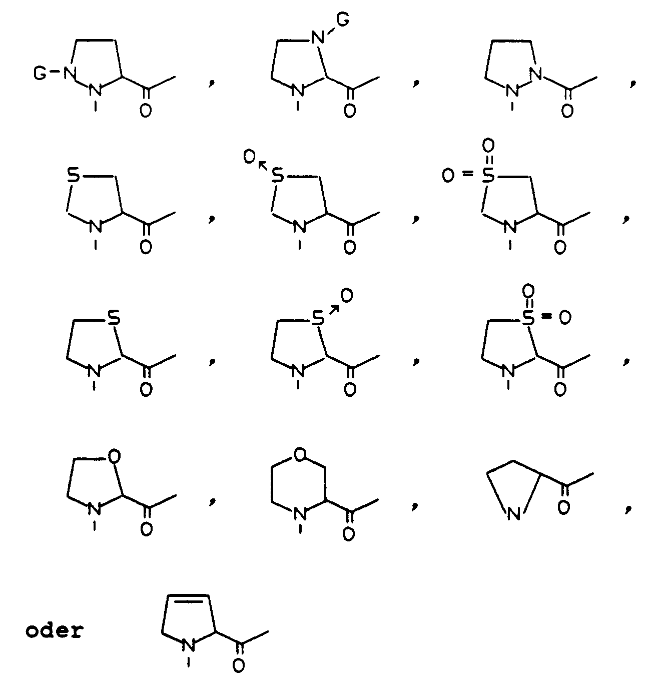 wobei G Wasserstoff bedeutet oder wie A unter a₁) oder a₂) definiert ist,
D Imino N-Methylimino,
Oxy oder
Methylen bedeutet,
E Carbonyl,
C=NR⁷,
C=N-OR⁷ oder
Sulfinyl bedeutet oder, falls F für eine Bindung steht, auch Hydroxymethylen bedeuten kann,
R⁷ Wasserstoff,
(C₁-C₈)-Alkyl oder
(C₃-C₈)-Cycloalkyl bedeutet,
F Oxy,
Imino,
N-Methylimino oder
eine direkte Bindung bedeutet,
R⁴ (C₁-C₆)-Alkyl,
(C₃-C₆)-Cycloalkyl,
(C₆-C₁₂)-Aryl,
(C₆-C₁₂)-Aryl-(C₁-C₅)-alkyl,
(C₆-C₁₂)-Aryloxy-(C₁-C₅)-alkyl,
(C₃-C₉)-Heteroaryl oder
(C₃-C₉)-Heteroaryl-(C₁-C₅)-alkyl bedeutet,
worin jeweils Alkyl gegebenenfalls durch 1 oder 2 gleiche oder verschiedene Reste aus der Reihe
Carboxy,
Amino,
(C₁-C₄)-Alkylamino,
Hydroxy,
(C₁-C₄)-Alkoxy,
Halogen,
Di-(C₁-C₄)-alkylamino,
Carbamoyl,
Sulfamoyl,
(C₁-C₄)-Alkoxycarbonyl,
(C₆-C₁₂)-Aryl und
(C₆-C₁₂)-Aryl-(C₁-C₅)-alkyl substituiert ist, und worin jeweils (C₆-C₁₂)-Aryl bzw. (C₃-C₉)-Heteroaryl gegebenenfalls durch 1, 2 oder 3 gleiche oder verschiedene Reste aus der Reihe
Carboxy,
Cyano,
Amino,
Nitro,
(C₁-C₄)-Alkylamino,
Hydroxy,
(C₁-C₄)-Alkoxy,
Halogen,
Di-(C₁-C₄)-alkylamino,
Carbamoyl,
Sulfamoyl und
(C₁-C₄)-Alkoxycarbonyl substituiert ist,
und
R⁸ wie R³ unter c₁) definiert ist,
sowie deren physiologisch verträgliche Salze, dadurch gekennzeichnet, daß man die Verbindung in bekannter Weise aus den Fragmenten beispielsweise durch deren Kupplung aufbaut, gegebenenfalls eine oder mehrere temporär eingeführte Schutzgruppe(n) abspaltet, Carbonylfunktionen gegebenenfalls in die Thia-Analogen überführt und die so erhaltene Verbindung der Formel I gegebenenfalls in ihr physiologisch verträgliches Salz überführt.

2. Verfahren gemäß Anspruch 1, dadurch gekennzeichnet, daß A gegebenenfalls substituiertes (C₁-C₈)-Alkyl, (C₁-C₈)-Alkanoyl oder gegebenenfalls substituiertes (C₁-C₈)-Alkoxycarbonyl bedeutet oder definiert ist, wie in Anspruch 1 unter a₃).

3. Verfahren gemäß Anspruch 1 und/oder 2, dadurch gekennzeichnet, daß R⁸ Wasserstoff bedeutet.

4. Verfahren gemäß einem oder mehreren der Ansprüche 1 bis 3, dadurch gekennzeichnet, daß Cyc 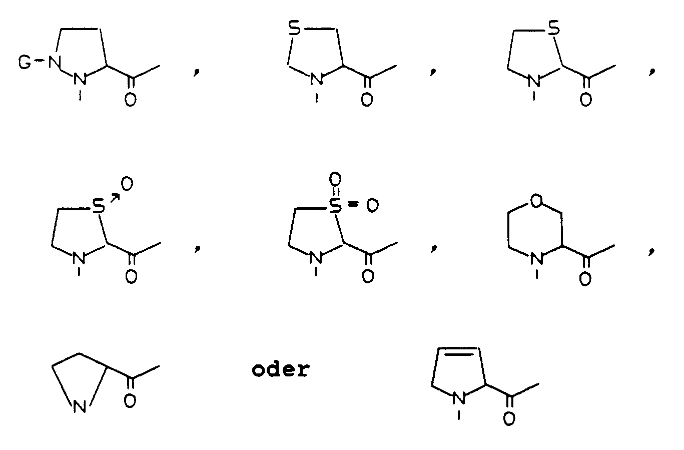 worin G für Wasserstoff steht oder wie A unter a₁) oder a₂) in Anspruch 1 definiert ist, bedeutet.

5. Verfahren gemäß einem oder mehreren der Ansprüche 1 bis 4, dadurch gekennzeichnet, daß D Imino, Oxy oder Methylen bedeutet.

6. Verfahren gemäß einem oder mehreren der Ansprüche 1 bis 5, dadurch gekennzeichnet, daß E Carbonyl bedeutet.

7. Verfahren gemäß einem oder mehreren der Ansprüche 1 bis 6, dadurch gekennzeichnet, daß F Oxy oder eine direkte Bindung bedeutet.

8. Verfahren gemäß einem oder mehreren der Ansprüche 1 bis 7, dadurch gekennzeichnet, daß R⁴ gegebenenfalls substituiertes (C₆-C₁₂)-Aryl oder gegebenenfalls im Alkylteil und/oder gegebenenfalls im Arylteil substituiertes (C₆-C₁₂)-Aryl-(C₁-C₅)-alkyl bedeutet.

9. Verwendung einer nach einem der Verfahren der Ansprüche 1 bis 8 hergestellten Verbindung der Formel I als Heilmittel.

10. Verwendung einer nach einem der Verfahren der Ansprüche 1 bis 8 hergestellten Verbindung der Formel I als Inhibitor der Prolylhydroxylase.

11. Verfahren zur Herstellung einer pharmazeutischen Zubereitung enthaltend eine nach einem der Verfahren der Ansprüche 1 bis 8 hergestellten Verbindung der Formel I, dadurch gekennzeichnet, daß man diese zusammen mit einem physiologisch unbedenklichen Träger und gegebenenfalls weiterem Hilfs- udn Zusatzstoffen in eine geeignete Darreichungsform bringt.

## Claims (Claims for the following Contracting State(s): AT, BE, CH, DE, FR, GB, IT, LI, LU, NL, SE)

1. A compound of the formula I 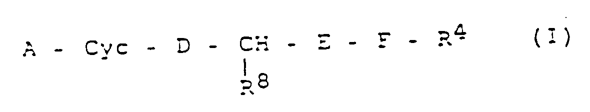 in which
A
a₁)
denotes (C₁-C₈)-alkyl,
(C₁-C₈)-alkanoyl,
(C₁-C₈)-alkoxycarbonyl or
(C₁-C₈)-alkylsulfonyl,
in which in each case 1, 2 or 3 hydrogen atoms are optionally replaced by 1, 2 or 3 identical or different radicals from the series comprising
carboxyl,
amino,
(C₁-C₄)-alkylamino,
hydroxyl,
(C₁-C₄)-alkoxy
halogen,
di-(C₁-C₄)-alkylamino,
carbamoyl,
sulfamoyl,
(C₁-C₄)-alkoxycarbonyl,
(C₆-C₁₂)-aryl and
(C₆-C₁₂)-aryl-(C₁-C₅)-alkyl,
or in which in each case 1 hydrogen atom is optionally replaced by a radical from the series comprising
(C₃-C₈)-cycloalkyl,
(C₁-C₄)-alkylsulfonyl,
(C₁-C₄)-alkylsulfinyl,
(C₆-C₁₂)-aryl-(C₁-C₄)-alkylsulfonyl,
(C₆-C₁₂)-aryl-(C₁-C₄)-alkylsulfinyl
(C₆-C₁₂)-aryloxy,
(C₃-C₉)-heteroaryl and
(C₃-C₉)-heteroaryloxy and
1 or 2 hydrogen atoms are replaced by 1 or 2 identical or different radicals from the series comprising
carboxyl,
amino,
(C₁-C₄)-alkylamino,
hydroxyl,
(C₁-C₄)-alkoxy,
halogen,
di-(C₁-C₄)-alkylamino,
carbamoyl,
sulfamoyl,
(C₁-C₄)-alkoxycarbonyl,
(C₆-C₁₂)-aryl and
(C₆-C₁₂)-aryl-(C₁-C₅)-alkyl,
a₂)
denotes (C₃-C₈)-cycloalkyl,
(C₆-C₁₂)-aryl,
(C₆-C₁₂)-arylsulfonyl or
(C₃-C₉)-heteroaryl,
where, in the radicals defined under a₁) and a₂), in each case (C₆-C₁₂)-aryl or (C₃-C₉)-heteroaryl is optionally substituted by 1, 2 or 3 identical or different radicals from the series comprising
carboxyl,
amino,
nitro,
(C₁-C₄)-alkylamino,
hydroxyl,
(C₁-C₄)-alkoxy,
halogen,
cyano,
di-(C₁-C₄)-alkylamino,
carbamoyl,
sulfamoyl and
(C₁-C₄)-alkoxycarbonyl,
or
a₃)
denotes a radical of the formula IIa or IIb 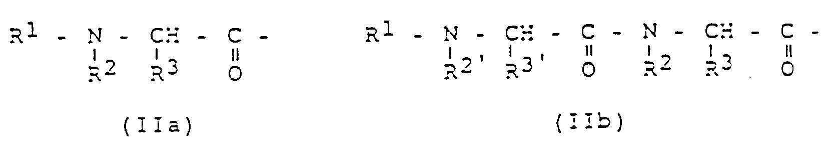
R₁
b₁)
denotes hydrogen or
b₂)
is defined as A under a₁) or a₂),
c₁)
R² and R^{2'} are identical or different and denote hydrogen or methyl,
R³ and R^{3'} are identical or different and denote hydrogen or (C₁-C₆)-alkyl, preferably (C₁-C₄)-alkyl, which is optionally monosubstituted by
amino,
benzyloxycarbonylamino,
hydroxyl,
carboxyl,
carbamoyl,
guanidino,
ureido,
mercapto,
methylmercapto,
phenyl,
4-chlorophenyl,
4-fluorophenyl,
4-nitrophenyl,
4-methoxyphenyl,
4-hydroxyphenyl,
phthalimido,
4-imidazolyl,
3-indolyl,
2-thienyl,
3-thienyl,
2-pyridyl,
3-pyridyl or
cyclohexyl,
c₂)
R² and R³ and/or R^{2'} and R^{3'} in each case together represent a [-CH₂-CH₂-CH₂-] chain in which one CH₂ group can be replaced by O, or
c₃)
R² and R³ and/or R^{2'} and R^{3'} in each case together represent 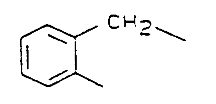 Cyc corresponds to one of the following heterocyclic rings, such as 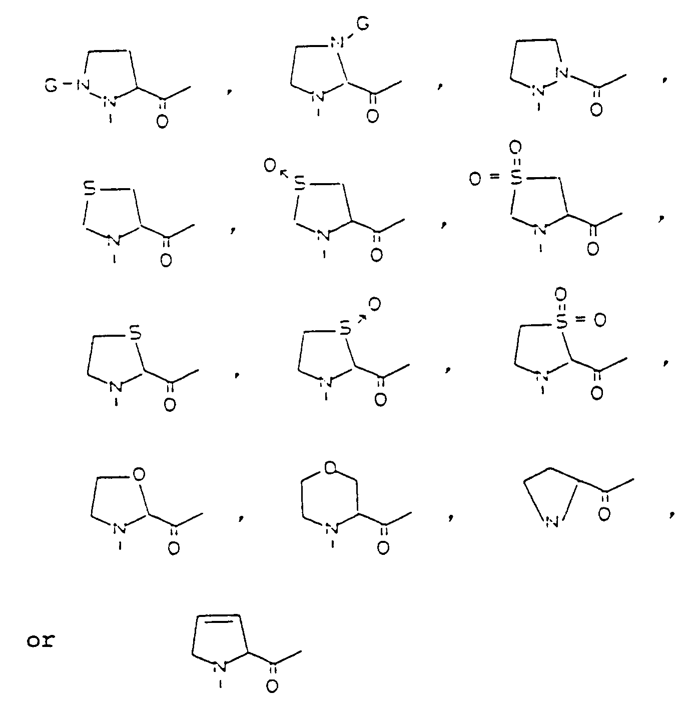 where G denotes hydrogen or is defined as A under a₁) or a₂),
D denotes imino,
N-methylimino,
oxy or
methylene,
E denotes carbonyl,
C=NR⁷,
C=N-OR' or
sulfinyl, or can also denote hydroxymethylene if F represents a bond,
R⁷ denotes hydrogen,
(C₁-C₈)-alkyl or
(C₃-C₈)-cycloalkyl,
F denotes oxy,
imino,
N-methylimino or
a direct bond,
R⁴ denotes (C₁-C₆)-alkyl,
(C₃-C₆)-cycloalkyl,
(C₆-C₁₂)-aryl,
(C₆-C₁₂)-aryl-(C₁-C₅)-alkyl,
(C₆-C₁₂)-aryloxy-(C₁-C₅)-alkyl,
(C₃-C₉)-heteroaryl or
(C₃-C₉)-heteroaryl-(C₁-C₅)-alkyl,
in which in each case alkyl is optionally substituted by 1 or 2 identical or different radicals from the series comprising
carboxyl,
amino,
(C₁-C₄)-alkylamino,
hydroxyl,
(C₁-C₄)-alkoxy,
halogen,
di-(C₁-C₄)-alkylamino,
carbamoyl,
sulfamoyl,
(C₁-C₄)-alkoxycarbonyl,
(C₆-C₁₂)-aryl and
(C₆-C₁₂)-aryl-(C₁-C₅)-alkyl,
and in which in each case (C₆-C₁₂)-aryl or (C₃-C₉)-heteroaryl is optionally substituted by 1, 2 or 3 identical or different radicals from the series comprising
carboxyl,
cyano,
amino,
nitro,
(C₁-C₄)-alkylamino,
hydroxyl,
(C₁-C₄)-alkoxy,
halogen,
di-(C₁-C₄)-alkylamino,
carbamoyl,
sulfamoyl and
(C₁-C₄)-alkyoxycarbonyl,
and
R⁸ is defined as R³ under c₁),
as well as the physiologically tolerated salts thereof.

2. A compound of the formula I as claimed in claim 1, in which A denotes optionally substituted (C₁-C₈)-alkyl, (C₁-C₈)-alkanoyl or optionally substituted (C₁-C₈)-alkoxycarbonyl, or is defined as in claim 1 under a₃), as well as the physiologically tolerated salts thereof.

3. A compound of the formula I as claimed in one or more of claims 1 to 2, in which R⁸ denotes hydrogen, as well as the physiologically tolerated salts thereof.

4. A compound of the formula I as claimed in one or more of claims 1 to 3, in which Cyc denotes 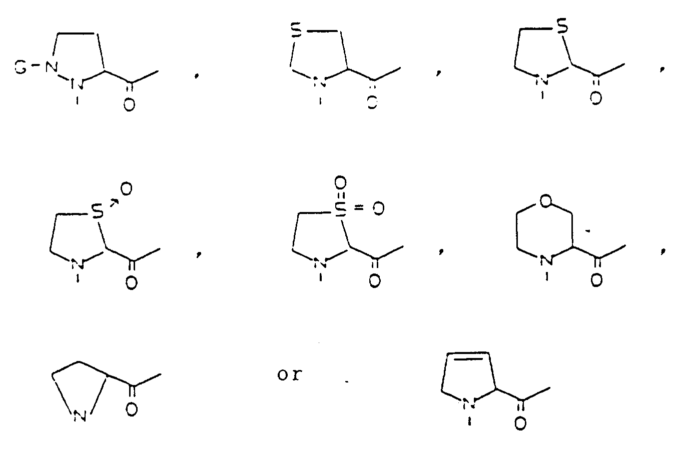 in which G represents hydrogen or is defined as A under a₁) or a₂) in claim 1, as well as the physiologically tolerated salts thereof.

5. A compound of the formula I as claimed in one or more of claims 1 to 4, in which D denotes imino, oxy or methylene, as well as the physiologically tolerated salts thereof.

6. A compound of the formula I as claimed in one or more of claims 1 to 5, in which E denotes carbonyl, as well as the physiologically tolerated salts thereof.

7. A compound of the formula I as claimed in one or more of claims 1 to 6, in which F denotes oxy or a direct bond, as well as the physiologically tolerated salts thereof.

8. A compound of the formula I as claimed in one or more of claims 1 to 7, in which R⁴ denotes optionally substituted (C₆-C₁₂)-aryl, or (C₆-C₁₂)-aryl-(C₁-C₅)-alkyl which is optionally substituted in the alkyl moiety and/or optionally substituted in the aryl moiety, as well as the physiologically tolerated salts thereof.

9. A process for the preparation of a compound of the formula I, which comprises building up the compound in a known manner from the fragments, for example by coupling thereof, where appropriate eliminating one or more temporarily introduced protective group(s), converting carbonyl groups into the thia analogs where appropriate, and converting the compound of the formula I which has been obtained in this way into a physiologically tolerated salt thereof where appropriate.

10. The use of a compound of the formula I as claimed in one or more of claims 1 to 8 as a medicine.

11. The use of a compound of the formula I as claimed in one or more of claims 1 to 8 as an inhibitor of prolyl hydroxylase.

12. A pharmaceutical composition containing a compound of the formula I as claimed in one or more of claims 1 to 8 and a physiologically acceptable vehicle.

## Claims (Claims for the following Contracting State(s): ES, GR)

1. A process for the preparation of a compound of the formula I 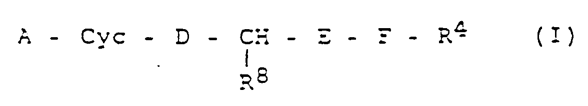 in which
A
a₁)
denotes (C₁-C₈)-alkyl,
(C₁-C₈)-alkanoyl,
(C₁-C₈)-alkoxycarbonyl or
(C₁-C₈)-alkylsulfonyl,
in which in each case 1, 2 or 3 hydrogen atoms are optionally replaced by 1, 2 or 3 identical or different radicals from the series comprising
carboxyl,
amino,
(C₁-C₄)-alkylamino,
hydroxyl,
(C₁-C₄)-alkoxy
halogen,
di-(C₁-C₄)-alkylamino,
carbamoyl,
sulfamoyl,
(C₁-C₄)-alkoxycarbonyl,
(C₆-C₁₂)-aryl and
(C₆-C₁₂)-aryl-(C₁-C₅)-alkyl,
or in which in each case 1 hydrogen atom is optionally replaced by a radical from the series comprising
(C₃-C₈)-cycloalkyl,
(C₁-C₄)-alkylsulfonyl,
(C₁-C₄)-alkylsulfinyl,
(C₆-C₁₂)-aryl-(C₁-C₄)-alkylsulfonyl,
(C₆-C₁₂)-aryl-(C₁-C₄)-alkylsulfinyl
(C₆-C₁₂)-aryloxy,
(C₃-C₉)-heteroaryl and
(C₃-C₉)-heteroaryloxy and
1 or 2 hydrogen atoms are replaced by 1 or 2 identical or different radicals from the series comprising
carboxyl,
amino,
(C₁-C₄)-alkylamino,
hydroxyl,
(C₁-C₄)-alkoxy,
halogen,
di-(C₁-C₄)-alkylamino,
carbamoyl,
sulfamoyl,
(C₁-C₄)-alkoxycarbonyl,
(C₆-C₁₂)-aryl and
(C₆-C₁₂)-aryl-(C₁-C₅)-alkyl,
a₂)
denotes (C₃-C₈)-cycloalkyl,
(C₆-C₁₂)-aryl,
(C₆-C₁₂)-arylsulfonyl or
(C₃-C₉)-heteroaryl,
where, in the radicals defined under a₁) and a₂), in each case (C₆-C₁₂)-aryl or (C₃-C₉)-heteroaryl is optionally substituted by 1, 2 or 3 identical or different radicals from the series comprising
carboxyl,
amino,
nitro,
(C₁-C₄)-alkylamino,
hydroxyl,
(C₁-C₄)-alkoxy,
halogen,
cyano,
di-(C₁-C₄)-alkylamino,
carbamoyl,
sulfamoyl and
(C₁-C₄)-alkoxycarbonyl,
or
a₃)
denotes a radical of the formula IIa or IIb 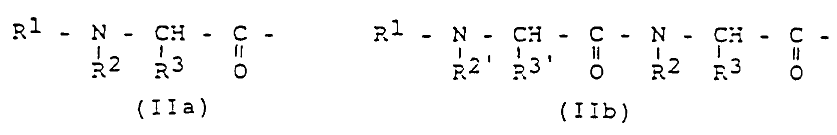
R¹
b₁)
denotes hydrogen or
b₂)
is defined as A under a₁) or a₂),
c₁)
R² and R^{2'} are identical or different and denote hydrogen or methyl,
R³ and R^{3'} are identical or different and denote hydrogen or (C₁-C₆)-alkyl, preferably (C₁-C₄)-alkyl, which is optionally monosubstituted by amino,
benzyloxycarbonylamino,
hydroxyl,
carboxyl,
carbamoyl,
guanidino,
ureido,
mercapto,
methylmercapto,
phenyl,
4-chlorophenyl,
4-fluorophenyl,
4-nitrophenyl,
4-methoxyphenyl,
4-hydroxyphenyl,
phthalimido,
4-imidazolyl,
3-indolyl,
2-thienyl,
3-thienyl,
2-pyridyl,
3-pyridyl or
cyclohexyl,
c₂)
R² and R³ and/or R^{2'} and R^{3'} in each case together represent a [-CH₂-CH₂-CH₂-] chain in which one CH₂ group can be replaced by O, or
c₃)
R² and R³ and/or R^{2'} and R^{3'} in each case together represent 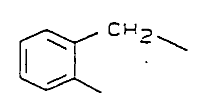 Cyc corresponds to one of the following heterocyclic rings, such as 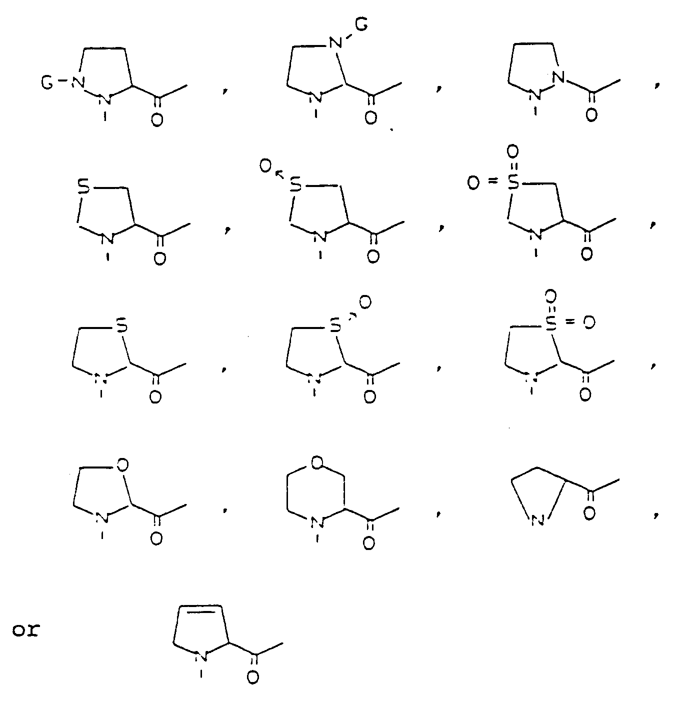 where G denotes hydrogen or is defined as A under a₁) or a₂),
D denotes imino,
N-methylimino,
oxy or
methylene,
E denotes carbonyl,
C=NR⁷,
C=N-OR⁷ or
sulfinyl, or can also denote hydroxymethylene if F represents a bond,
R₇ denotes hydrogen,
(C₁-C₈)-alkyl or
(C₃-C₈)-cycloalkyl,
F denotes oxy,
imino,
N-methylimino or a direct bond,
R⁴ denotes (C₁-C₆)-alkyl,
(C₃-C₆)-cycloalkyl,
(C₆-C₁₂)-aryl,
(C₆-C₁₂)-aryl-(C₁-C₅)-alkyl,
(C₆-C₁₂)-aryloxy-(C₁-C₅)-alkyl,
(C₃-C₉)-heteroaryl or
(C₃-C₉)-heteroaryl-(C₁-C₅)-alkyl,
in which in each case alkyl is optionally substituted by 1 or 2 identical or different radicals from the series comprising
carboxyl,
amino,
(C₁-C₄)-alkylamino,
hydroxyl,
(C₁-C₄)-alkoxy,
halogen,
di-(C₁-C₄)-alkylamino,
carbamoyl,
sulfamoyl,
(C₁-C₄)-alkoxycarbonyl,
(C₆-C₁₂)-aryl and
(C₆-C₁₂)-aryl-(C₁-C₅)-alkyl,
and in which in each case (C₆-C₁₂)-aryl or (C₃-C₉)-heteroaryl is optionally substituted by 1, 2 or 3 identical or different radicals from the series comprising
carboxyl,
cyano,
amino,
nitro,
(C₁-C₄)-alkylamino,
hydroxyl,
(C₁-C₄)-alkoxy,
halogen,
di-(C₁-C₄)-alkylamino,
carbamoyl,
sulfamoyl and
(C₁-C₄)-alkyoxycarbonyl,
and
R⁸ is defined as R³ under c₁),
as well as the physiologically tolerated salts thereof, which comprises building up the compound in a known manner from the fragments, for example by coupling thereof, where appropriate eliminating one or more temporarily introduced protective group(s), converting carbonyl groups into the thia analogs where appropriate, and converting the compound of the formula I which has been obtained in this way into a physiologically tolerated salt thereof where appropriate.

2. The process as claimed in claim 1, wherein A denotes optionally substituted (C₁-C₈)-alkyl, (C₁-C₈)-alkanoyl or optionally substituted (C₁-C₈)-alkoxycarbonyl, or is defined as in claim 1 under a₃).

3. The process as claimed in claim 1 and/or 2, wherein R⁸ denotes hydrogen.

4. The process as claimed in one or more of claims 1 to 3, wherein Cyc denotes 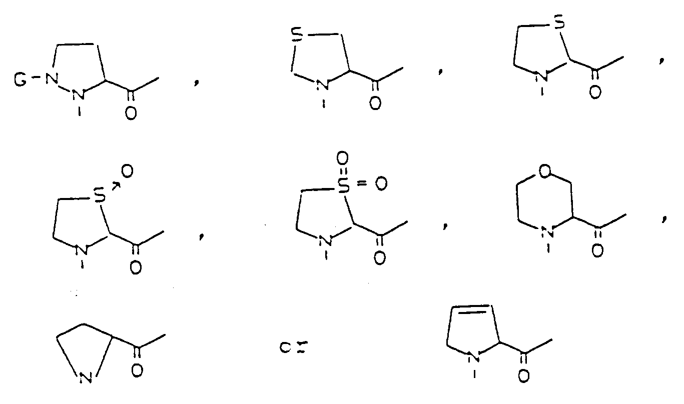 in which G represents hydrogen or is defined as A under a₁) or a₂) in claim 1.

5. The process as claimed in one or more of claims 1 to 4, wherein D denotes imino, oxy or methylene.

6. The process as claimed in one or more of claims 1 to 5, wherein E denotes carbonyl.

7. The process as claimed in one or more of claims 1 to 6, wherein F denotes oxy or a direct bond.

8. The process as claimed in one or more of claims 1 to 7, wherein R⁴ denotes optionally substituted (C₆-C₁₂)-aryl, or (C₆-C₁₂)-aryl-(C₁-C₅)-alkyl which is optionally substituted in the alkyl moiety and/or optionally substituted in the aryl moiety.

9. The use of a compound of the formula I prepared by one of the processes of claims 1 to 8 as a medicine.

10. The use of a compound of the formula I prepared by one of the processes of claims 1 to 8 as an inhibitor of prolyl hydroxylase.

11. A process for the preparation of a pharmaceutical composition containing a compound of the formula I prepared by one of the processes of claims 1 to 8, which comprises converting this compound together with a physiologically acceptable vehicle and, where appropriate, further auxiliaries and additives into a suitable dosage form.

## Revendications (Revendications pour l'(les) Etat(s) contractant(s) suivant(s): AT, BE, CH, DE, FR, GB, IT, LI, LU, NL, SE)

1. Composés de formule I 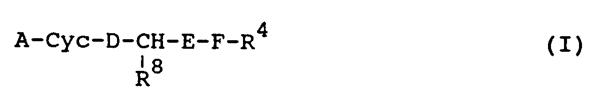 dans laquelle
A représente
a₁)
un groupe alkyle en C₁-C₈,
alcanoyle en C₁-C₈,
(alcoxy en C₁-C₈)-carbonyle ou
(alkyl en C₁-C₈)-sulfonyle,
dans lesquels 1, 2 ou 3 atomes d'hydrogène sont dans chaque cas éventuellement remplacés par 1, 2 ou 3 radicaux, identiques ou différents, choisis parmi
des radicaux carboxy,
amino,
(alkyl en C₁-C₄)-amino,
hydroxy, alcoxy en C₁-C₄,
des atomes d'halogène,
des radicaux di(alkyl en C₁-C₄)-amino,
carbamoyle,
sulfamoyle,
(alcoxy en C₁-C₄)-carbonyle,
aryle en C₆-C₁₂ et
(aryl en C₆-C₁₂)-alkyle en C₁-C₅, ou
dans lesquels dans chaque cas un atome d'hydrogène est éventuellement remplacé par un radical choisi parmi
des radicaux cycloalkyle en C₃-C₈,
(alkyl en C₁-C₄)-sulfonyle,
(alkyl en C₁-C₄)-sulfinyle,
(aryl en C₆-C₁₂)-(alkyl en C₁-C₄)-sulfonyle,
(aryl en C₆-C₁₂)-(alkyl en C₁-C₄)-
sulfinyle,
aryloxy en C₆-C₁₂,
hétéroaryle en C₃-C₉ et
hétéroaryloxy en C₃-C₉,
et
1 ou 2 atomes d'hydrogène sont remplacés par 1 ou 2 radicaux, identiques ou différents, choisis parmi
des radicaux carboxy,
amino,
(alkyl en C₁-C₄)-amino,
hydroxy,
alcoxy en C₁-C₄,
des atomes d'halogène,
des radicaux di(alkyl en C₁-C₄)-amino,
carbamoyle,
sulfamoyle,
(alcoxy en C₁-C₄)-carbonyle,
aryle en C₆-C₁₂ et
(aryl en C₆-C₁₂)-alkyle en C₁-C₅,
a₂)
un groupe cycloalkyle en C₃-C₈,
aryle en C₆-C₁₂,
(aryl en C₆-C₁₂)-sulfonyle, ou
hétéroaryle en C₃-C₉,
dans les radicaux définis en a₁) et a₂), un radical aryle en C₆-C₁₂ ou hétéroaryle en C₃-C₉ étant dans chaque cas éventuellement substitué par 1, 2 ou 3 radicaux identiques ou différents, choisis parmi
des radicaux carboxy,
amino,
nitro,
(alkyl en C₁-C₄)-amino,
hydroxy,
alcoxy en C₁-C₄,
des atomes d'halogène,
des radicaux cyano,
di(alkyl en C₁-C₄)-amino,
carbamoyle,
sulfamoyle, et
(alcoxy en C₁-C₄)-carbonyle, ou
a₃)
un radical de formule IIa ou IIb 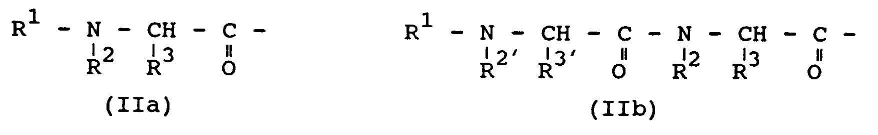
R¹
b₁)
représente un atome d'hydrogène ou
b₂)
est tel que A défini en a₁) ou a₂),
c₁)
R² et R^{2'} sont identiques ou différents et représentent l'atome d'hydrogène ou le groupe méthyle,
R³ et R^{3'} sont identiques ou différents et représentent un atome d'hydrogène ou
un groupe alkyle en C₁-C₆, de préférence alkyle en C₁-C₄,
qui est éventuellement monosubstitué par
un radical amino,
benzyloxycarbonylamino,
hydroxy,
carboxy,
carbamoyle,
guanidino,
uréido,
mercapto,
méthylmercapto,
phényle,
4-chlorophényle,
4-fluorophényle,
4-nitrophényle,
4-méthoxyphényle,
4-hydroxyphényle,
phtalimido,
4-imidazolyle,
3-indolyle,
2-thiényle,
3-thiényle,
2-pyridyle,
3-pyridyle, ou
cyclohexyle,
c₂)
R² et R³ et/ou R^{2'} et R^{3'} , respectivement, représentent ensemble une chaîne (-CH₂-CH₂-CH₂-), dans laquelle un groupe CH₂ peut être remplacé par O, ou
c₃)
R² et R³ et/ou R^{2'} et R^{3'}, respectivement, représentent ensemble 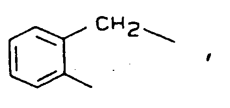 Cyc correspond à l'un des hétérocycles suivants, tels que 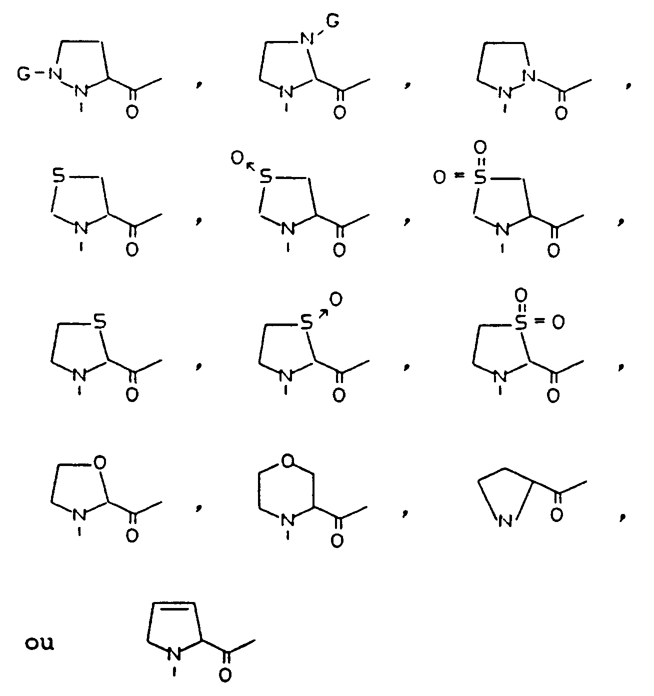 G représentant un atome d'hydrogène ou ayant la même définition que A en a₁) ou a₂),
D représente le groupe imino,
N-méthylimino,
oxy ou
méthylène,
E représente un groupe carbonyle,
C=NR⁷,
C=N-OR⁷ ou
sulfinyle ou, lorsque F représente une liaison, peut également représenter le groupe hydroxyméthylène,
R⁷ représente un atome d'hydrogène,
un groupe alkyle en C₁-C₈ ou
cycloalkyle en C₃-C₈,
F représente le groupe oxy,
imino,
N-méthylimino ou
une liaison directe,
R⁴ représente un groupe alkyle en C₁-C₆,
cycloalkyle en C₃-C₆,
aryle en C₆-C₁₂,
(aryl en C₆-C₁₂)-alkyle en C₁-C₅,
(aryloxy en C₆-C₁₂)-alkyle en C₁-C₅,
hétéroaryle en C₃-C₉ ou
(hétéroaryl en C₃-C₉)-alkyle en C₁-C₅, chaque fragment alkyle étant éventuellement substitué par 1 ou 2 radicaux identiques ou différents, choisis parmi
des radicaux carboxy,
amino,
(alkyl en C₁-C₄)-amino,
hydroxy,
alcoxy en C₁-C₄,
des atomes d'halogène,
des radicaux di(alkyl en C₁-C₄)-amino,
carbamoyle,
sulfamoyle,
(alcoxy en C₁-C₄)-carbonyle,
aryle en C₆-C₁₂ et
(aryl en C₆-C₁₂)-alkyle en C₁-C₅,
et dans lesquels chaque fragment aryle en C₆-C₁₂ ou hétéroaryle en C₃-C₉ est éventuellement substitué par 1, 2 ou 3 radicaux identiques ou différents, choisis parmi
des radicaux carboxy,
cyano,
amino,
nitro,
(alkyl en C₁-C₄)-amino,
hydroxy,
alcoxy en C₁-C₄,
des atomes d'halogène,
des radicaux di(alkyl en C₁-C₄)-amino,
carbamoyle,
sulfamoyle et
(alcoxy en C₁-C₄)-carbonyle, et
R⁸ est défini comme R³ en c₁),
et sels physiologiquement acceptables de ceux-ci.

2. Composé de formule I selon la revendication 1, dans lequel A représente un groupe alkyle en C₁-C₈ éventuellement substitué, alcanoyle en C₁-C₈ ou (alcoxy en C₁-C₈)-carbonyle éventuellement substitué ou est défini comme dans la revendication 1 en a₃), et sels physiologiquement acceptables de celui-ci.

3. Composé de formule I selon une ou plusieurs des revendications 1 et 2, dans lequel R⁸ représente un atome d'hydrogène, et sels physiologiquement acceptables de celui-ci.

4. Composé de formule I selon une ou plusieurs des revendications 1 à 3, dans lequel Cyc représente 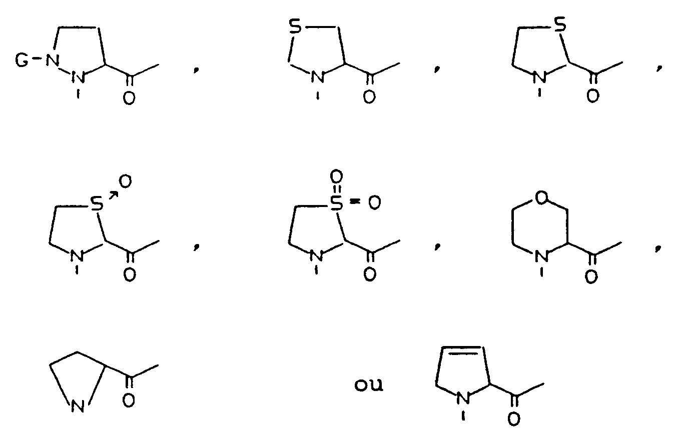 G représentant un atome d'hydrogène ou étant défini comme A en a₁) ou a₂) dans la revendication 1, et sels physiologiquement acceptables de celui-ci.

5. Composé de formule I selon une ou plusieurs des revendications 1 à 4, dans lequel D représente le groupe imino, oxy ou méthylène, et sels physiologiquement acceptables de celui-ci.

6. Composé de formule I selon une ou plusieurs des revendications 1 à 5, dans lequel E représente le groupe carbonyle, et sels physiologiquement acceptables de celui-ci.

7. Composé de formule I selon une ou plusieurs des revendications 1 à 6, dans lequel F représente le groupe oxy ou une liaison directe, et sels physiologiquement acceptables de celui-ci.

8. Composé de formule I selon une ou plusieurs des revendications 1 à 7, dans lequel
R⁴ représente un groupe aryle en C₆-C₁₂ éventuellement substitué ou un groupe (aryl en C₆-C₁₂)-alkyle en C₁-C₅ éventuellement substitué sur le fragment alkyle et/ou éventuellement substitué sur le fragment aryle, et sels physiologiquement acceptables de celui-ci.

9. Procédé pour la préparation d'un composé de formule I, caractérisé en ce que l'on fait la synthèse du composé, de façon connue, à partir des fragments, par exemple par couplage de ceux-ci, éventuellement on élimine un ou plusieurs groupe(s) protecteur(s) temporairement introduit(s), éventuellement on convertit des fonctions carbonyle en les analogues thia, et le composé de formule I ainsi obtenu est éventuellement converti en un de ses sels physiologiquement acceptables.

10. Utilisation d'un composé de formule I selon une ou plusieurs des revendications 1 à 8, en tant que médicament.

11. Utilisation d'un composé de formule I selon une ou plusieurs des revendications 1 à 8, en tant qu'inhibiteur de la prolylhydroxylase.

12. Composition pharmaceutique contenant un composé de formule I selon une ou plusieurs des revendications 1 à 8 et un véhicule physiologiquement acceptable.

## Revendications (Revendications pour l'(les) Etat(s) contractant(s) suivant(s): ES, GR)

1. Procédé pour la préparation d'un composé de formule I 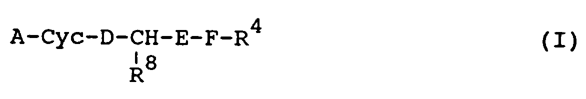 dans laquelle
A représente
a₁)
un groupe alkyle en C₁-C₈,
alcanoyle en C₁-C₈,
(alcoxy en C₁-C₈)-carbonyle ou
(alkyl en C₁-C₈)-sulfonyle,
dans lesquels 1, 2 ou 3 atomes d'hydrogène sont dans chaque cas éventuellement remplacés par 1, 2 ou 3 radicaux, identiques ou différents, choisis parmi
des radicaux carboxy,
amino,
(alkyl en C₁-C₄)-amino,
hydroxy, alcoxy en C₁-C₄,
des atomes d'halogène,
des radicaux di(alkyl en C₁-C₄)-amino,
carbamoyle,
sulfamoyle,
(alcoxy en C₁-C₄)-carbonyle,
aryle en C₆-C₁₂ et
(aryl en C₆-C₁₂)-alkyle en C₁-C₅, ou dans lesquels dans chaque cas un atome d'hydrogène est éventuellement remplacé par un radical choisi parmi
des radicaux cycloalkyle en C₃-C₈,
(alkyl en C₁-C₄)-sulfonyle,
(alkyl en C₁-C₄)-sulfinyle,
(aryl en C₆-C₁₂)-(alkyl en C₁-C₄)-sulfonyle,
(aryl en C₆-C₁₂)-(alkyl en C₁-C₄)-
sulfinyle,
aryloxy en C₆-C₁₂,
hétéroaryle en C₃-C₉ et
hétéroaryloxy en C₃-C₉,
et
1 ou 2 atomes d'hydrogène sont remplacés par 1 ou 2 radicaux, identiques ou différents, choisis parmi
des radicaux carboxy,
amino,
(alkyl en C₁-C₄)-amino,
hydroxy,
alcoxy en C₁-C₄,
des atomes d'halogène,
des radicaux di(alkyl en C₁-C₄)-amino,
carbamoyle,
sulfamoyle,
(alcoxy en C₁-C₄)-carbonyle,
aryle en C₆-C₁₂ et
(aryl en C₆-C₁₂)-alkyle en C₁-C₅,
a₂)
un groupe cycloalkyle en C₃-C₈,
aryle en C₆-C₁₂,
(aryl en C₆-C₁₂)-sulfonyle, ou
hétéroaryle en C₃-C₉,
dans les radicaux définis en a₁) et a₂), un radical aryle en C₆-C₁₂ ou hétéroaryle en C₃-C₉ étant dans chaque cas éventuellement substitué par 1, 2 ou 3 radicaux identiques ou différents, choisis parmi
des radicaux carboxy,
amino,
nitro,
(alkyl en C₁-C₄)-amino,
hydroxy,
alcoxy en C₁-C₄,
des atomes d'halogène,
des radicaux cyano,
di(alkyl en C₁-C₄)-amino,
carbamoyle,
sulfamoyle, et
(alcoxy en C₁-C₄)-carbonyle, ou
a₃)
un radical de formule IIa ou IIb 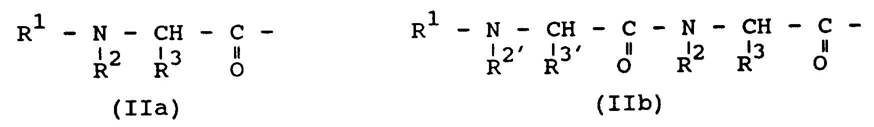
R¹
b₁)
représente un atome d'hydrogène ou
b₂)
est tel que A défini en a₁) ou a₂),
c₁)
R² et R^{2'} sont identiques ou différents et représentent l'atome d'hydrogène ou le groupe méthyle,
R³ et R^{3'} sont identiques ou différents et représentent un atome d'hydrogène ou
un groupe alkyle en C₁-C₆, de préférence
alkyle en C₁-C₄,
qui est éventuellement monosubstitué par
un radical amino,
benzyloxycarbonylamino,
hydroxy,
carboxy,
carbamoyle,
guanidino,
uréido,
mercapto,
méthylmercapto,
phényle,
4-chlorophényle,
4-fluorophényle,
4-nitrophényle,
4-méthoxyphényle,
4-hydroxyphényle,
phtalimido,
4-imidazolyle,
3-indolyle,
2-thiényle,
3-thiényle,
2-pyridyle,
3-pyridyle, ou
cyclohexyle,
c₂)
R² et R³ et/ou R^{2'} et R^{3'}, respectivement, représentent ensemble une chaîne (-CH₂-CH₂-CH₂-), dans laquelle un groupe CH₂ peut être remplacé par O, ou
c₃)
R² et R³ et/ou R^{2'} et R^{3'}_{,} respectivement, représentent ensemble 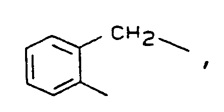 Cyc correspond à l'un des hétérocycles suivants, tels que 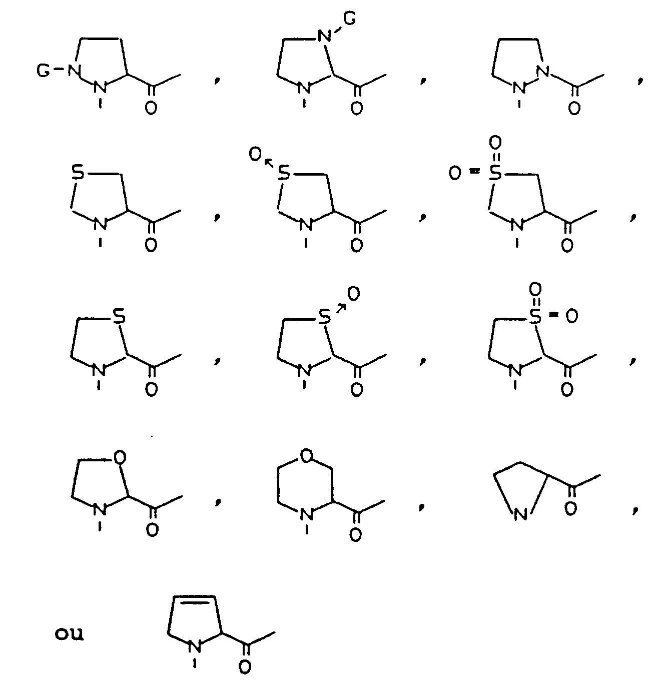 G représentant un atome d'hydrogène ou ayant la même définition que A en a₁) ou a₂),
D représente le groupe imino,
N-méthylimino,
oxy ou
méthylène,
E représente un groupe carbonyle,
C=NR⁷,
C=N-OR⁷ ou
sulfinyle ou, lorsque F représente une liaison, peut également représenter le groupe hydroxyméthylène,
R⁷ représente un atome d'hydrogène,
un groupe alkyle en C₁-C₈ ou
cycloalkyle en C₃-C₈,
F représente le groupe oxy,
imino,
N-méthylimino ou
une liaison directe,
R⁴ représente un groupe alkyle en C₁-C₆,
cycloalkyle en C₃-C₆,
aryle en C₆-C₁₂,
(aryl en C₆-C₁₂)-alkyle en C₁-C₅,
(aryloxy en C₆-C₁₂)-alkyle en C₁-C₅,
hétéroaryle en C₃-C₉ ou
(hétéroaryl en C₃-C₉)-alkyle en C₁-C₅,
chaque fragment alkyle étant éventuellement substitué par 1 ou 2 radicaux identiques ou différents, choisis parmi
des radicaux carboxy,
amino,
(alkyl en C₁-C₄)-amino,
hydroxy,
alcoxy en C₁-C₄,
des atomes d'halogène,
des radicaux di(alkyl en C₁-C₄)-amino,
carbamoyle,
sulfamoyle,
(alcoxy en C₁-C₄)-carbonyle,
aryle en C₆-C₁₂ et
(aryl en C₆-C₁₂)-alkyle en C₁-C₅,
et dans lesquels chaque fragment aryle en C₆-C₁₂ ou hétéroaryle en C₃-C₉ est éventuellement substitué par 1, 2 ou 3 radicaux identiques ou différents, choisis parmi
des radicaux carboxy,
cyano,
amino,
nitro,
(alkyl en C₁-C₄)-amino,
hydroxy,
alcoxy en C₁-C₄,
des atomes d'halogène,
des radicaux di(alkyl en C₁-C₄)-amino,
carbamoyle,
sulfamoyle et
(alcoxy en C₁-C₄)-carbonyle, et
R⁸ est défini comme R³ en c₁),
ainsi que de leurs sels physiologiquement acceptables, caractérisé en ce que l'on fait la synthèse du composé, de façon connue, à partir des fragments, par exemple par couplage de ceux-ci, éventuellement on élimine un ou plusieurs groupe(s) protecteur(s) temporairement introduit(s), éventuellement on convertit des fonctions carbonyle en les analogues thia, et le composé de formule I ainsi obtenu est éventuellement converti en un de ses sels physiologiquement acceptables.

2. Procédé selon la revendication 1, caractérisé en ce que A représente un groupe alkyle en C₁-C₈ éventuellement substitué, alcanoyle en C₁-C₈ ou (alcoxy en C₁-C₈)-carbonyle éventuellement substitué ou est défini comme dans la revendication 1 en a₃).

3. Procédé selon la revendication 1 et/ou la revendication 2, caractérisé en ce que R⁸ représente un atome d'hydrogène.

4. Procédé selon une ou plusieurs des revendications 1 à 3, caractérisé en ce que Cyc représente 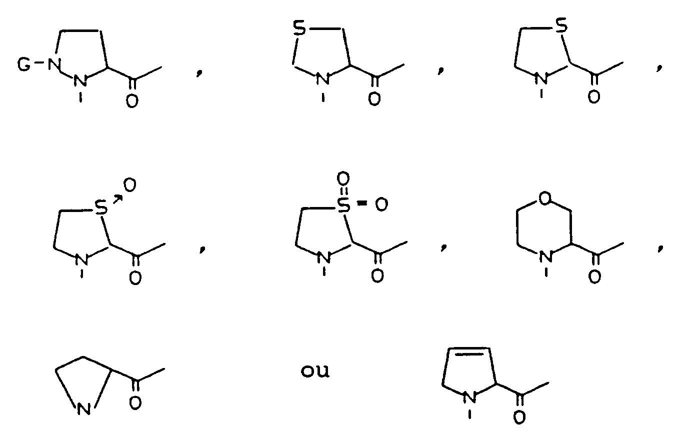 G représentant un atome d'hydrogène ou étant défini comme A en a₁) ou a₂) dans la revendication 1.

5. Procédé selon une ou plusieurs des revendications 1 à 4, caractérisé en ce que D représente le groupe imino, oxy ou méthylène.

6. Procédé selon une ou plusieurs des revendications 1 à 5, caractérisé en ce que E représente le groupe carbonyle.

7. Procédé selon une ou plusieurs des revendications 1 à 6, caractérisé en ce que F représente le groupe oxy ou une liaison directe.

8. Procédé selon une ou plusieurs des revendications 1 à 7, caractérisé en ce que R⁴ représente un groupe aryle en C₆-C₁₂ éventuellement substitué ou un groupe (aryl en C₆-C₁₂)-alkyle en C₁-C₅ éventuellement substitué sur le fragment alkyle et/ou éventuellement substitué sur le fragment aryle.

9. Utilisation d'un composé de formule I, préparé selon l'un des procédés des revendications 1 à 8, en tant que médicament.

10. Utilisation d'un composé de formule I, préparé selon l'un des procédés des revendications 1 à 8, en tant qu'inhibiteur de la prolylhydroxylase.

11. Procédé pour la préparation d'une composition pharmaceutique contenant un composé de formule I, préparé selon l'un des procédés des revendications 1 à 8, caractérisé en ce que l'on met celui-ci sous une forme pharmaceutique appropriée, conjointement avec un véhicule physiologiquement acceptable et éventuellement d'autres adjuvants et additifs.
